# EUROPEAN PATENT SPECIFICATION

(11) **EP 2 791 350 B1**
(45) Date of publication and mention of the grant of the patent: **23.01.2019**
(21) Application number: 12799484.6
(22) Date of filing: 04.12.2012
(51) Int. Cl.: C12P 39/00, C12P 7/06, C12P 7/08, C12P 7/10

(54) **ENZYME COCKTAILS PREPARED FROM MIXED CULTURES**
ENZYMCOCKTAILS AUS GEMISCHTEN KULTUREN
COCKTAILS ENZYMATIQUES PRÉPARÉS À PARTIR DE CULTURES MIXTES

(30) Priority: 13.12.2011 US 201161570243 P
(43) Date of publication of application: 22.10.2014
(73) Proprietor: Danisco US Inc., Palo Alto, California 94304 (US)
(72) Inventor: ENGLAND, George, Palo Alto, California 94304 (US); LANTZ, Suzanne E., Palo Alto, California 94304 (US)
(74) Representative: Mewburn Ellis LLP
(86) International application number: PCT/US2012/067717
(87) International publication number: WO 2013/090053

(56) References cited:
- WO-A2-2004/113490
- WO-A2-2010/060056
- WEN ZHIYOU ET AL: "Production of cellulase/beta-glucosidase by the mixed fungi culture of Trichoderma reesei and Aspergillus phoenicis on dairy manure", APPLIED BIOCHEMISTRY AND BIOTECHNOLOGY, vol. 121, April 2005 (2005-04), pages 93-104, XP002696851, ISSN: 0273-2289
- HOURIA OULED HADDAR ET AL: "Biodegradation of native feather keratin by Bacillus subtilis recombinant strains", BIODEGRADATION, vol. 20, no. 5, 26 March 2009 (2009-03-26) , pages 687-694, XP019727763, KLUWER ACADEMIC PUBLISHERS, DO ISSN: 1572-9729, DOI: 10.1007/S10532-009-9256-0
- RAJ KUMAR ET AL: "Bioconversion of lignocellulosic biomass: biochemical and molecular perspectives", JOURNAL OF INDUSTRIAL MICROBIOLOGY & BIOTECHNOLOGY ; OFFICIAL JOURNAL OF THE SOCIETY FOR INDUSTRIAL MICROBIOLOGY, vol. 35, no. 5, 13 March 2008 (2008-03-13) , pages 377-391, XP019596328, SPRINGER, BERLIN, DE ISSN: 1476-5535
- ARAI TAKAMITSU ET AL: "Synthesis of Clostridium cellulovorans minicellulosomes by intercellular complementation", PROCEEDINGS OF THE NATIONAL ACADEMY OF SCIENCES OF THE UNITED STATES OF AMERICA, vol. 104, no. 5, January 2007 (2007-01), pages 1456-1460, XP002696852, ISSN: 0027-8424
- Walton J, Banerjee G, Car S: "GENPLAT: an automated platform for biomass enzyme discovery and cocktail optimization.", Journal of Visualized Experiments , vol. 56, E3314, 24 October 2011 (2011-10-24), XP002696853, DOI: 10.3791/3314 Retrieved from the Internet: URL:http://www.jove.com/video/3314/genplat -an-automated-platform-for-biomass-enzyme- discovery-cocktail?id=3314 [retrieved on 2013-05-08]
- SRIVASTAVA S K ET AL: "The production of beta-glucosidase in shake-flasks by Aspergillus wentii", JOURNAL OF FERMENTATION TECHNOLOGY, ELSEVIER, JP, vol. 65, no. 1, 1 January 1987 (1987-01-01), pages 95-99, XP025764008, ISSN: 0385-6380, DOI: 10.1016/0385-6380(87)90071-9 [retrieved on 1987-01-01]
- MAHESHWARI D K ET AL: "Paper mill sludge as a potential source for cellulase production by Trichoderma reesei QM 9123 and Aspergillus niger using mixed cultivation", CARBOHYDRATE POLYMERS, APPLIED SCIENCE PUBLISHERS, LTD. BARKING, GB, vol. 23, no. 3, 1 January 1994 (1994-01-01), pages 161-163, XP024147532, ISSN: 0144-8617, DOI: 10.1016/0144-8617(94)90098-1 [retrieved on 1994-01-01]

## Description

### BACKGROUND OF THE INVENTION

Enzyme cocktails are used in many industrial processes. Enzyme cocktails can be prepared by individual production of enzymes in separate cell lines followed by blending. Individualized production and blending is often associated with substantial costs. Enzyme cocktails can also be prepared by engineering all desired enzymes for a particular cocktail into a single production cell line. This method lacks flexibility because in this case the enzymes expressed by a particular cell line are always produced in the same or nearly the same proportion. This lacking in flexibility is a drawback, especially when the enzyme cocktails produced by this method are for applications in processes such as biomass hydrolysis and grain processing. In these applications, the selection and performance of enzyme cocktails often depend on the type of substrates and/or pretreatment methods. A different blend may be needed for different substrates or pretreatments. A new production host may need to be built each time a new or even minutely modified blend is desired.

Developing bacterial co-cultures of cell lines has been reported as tedious because of different growth requirements (Dashtban et al., Int. J. of Biol. Sci., 5:578-595, 2009) for different cell lines. Co-establishment of a stable co-culture has been reported to depend on media and growth requirements, such as temperature, atmosphere and carbon source, (Maki et al., Int. J. of Biol. Sciences, 5:500-516, 2009). Co-cultures have been reported to be affected by metabolic interactions (i.e. syntrophic relationships or alternatively competition for substrates) and other interactions (i.e. growth promoting or growth inhibiting such as antibiotics) (see, e.g., Maki et al., Int. J. of Biol. Sciences, 5:500-516, 2009).

Solid state co-fermentation (e.g., using fermentation trays) of two fungal strains has been reported (*see, e.g.,* Sun et al., Electronic J. of Biotechnology, 12: 1-13, 2008; Pandey et al., Curr. Sci., 77:149-162, 1999; Hu et al., International Biodeterioration & Biodegradation 65:248-252, 2011; Wang et al., Appl. Microbiol. Biotechnol. 73:533-540, 2006). However, solid state co-fermentations are difficult and are not always suitable for recombinant production of enzymes at industrial scales. Submerged fermentations are often more flexible and deemed more desirable, which have been used on, for example, *Penicillium sp.* CH-TE-001 and *Aspergillus terreus* CH-TE-013 for producing an enzyme mixture (Garcia-Kirchner, et al., Applied Biochem & Biotechnol. 98:1105-1114, 2002). In addition, mixed cultures of microorganisms have been fermented under different conditions to obtain cultivated microorganisms enriched for certain characteristics, which are then blended to obtain a formulated complex culture (*see, e.g* EP 2292731).
Wen et al. (Process Biochemistry 2005; 40: 3087-3094) describe production of cellulase/β-glucosidase by the mixed fungi culture *Trichoderma reesei* and *Aspergillus phoenicis* on dairy manure.
Haddar et al (Biodegradation 2009; 20:687-694) describe biodegradation of native feather keratin by *Bacillus subtilis* recombinant strains.
WO 2010/060056 recites yeast expressing cellulases for simultaneous saccharification and fermentation using cellulose.
WO 2004/113490 recites a method for directed selective solid-phase culture of stable microbial mixed population for the continuous preparation of defined enzyme and metabolite mixtures, enzyme and metabolite materials obtained by such method and suitable devices thereof.
Kumar et al. (J Ind Microbiol Biotechnol 2008; 35(5): 377-381) describe biochemical and molecular perspectives to the bioconversion of lignocellulosic biomass, including co-cultivation of microbes.
Arai et al. (PNAS 2007; 104(5): 1456-1460) describe synthesis of Clostridium cellulovorans minicellulosomes by intercellular complementation.
Walton et al. (Journal of Visualized Experiments 2011: 56(e3315): 1-5) describe GENPLAT, an automated platform for biomass enzyme discovery and cocktail optimization.
Srivastava S K et al. (Journal of Fermentation Technology 1987; 65(1): 95-99) describe the production of β-glucosidase in shake-flasks by *Aspergillus wentii.*
Maheshwari D K et al. (Carbohydrate Polymers 1994; 3:161-163) describe paper mill sludge as a potential source for cellulase production for *Trichoderma reesei* QM 9123 and *Aspergillus niger* using mixed cultivation.

### BRIEF DESCRIPTION OF THE FIGURES

Figure 1: A pENTR/D-TOPO vector with the Fv3C open reading frame.
Figure 2: A map of the expression plasmid pTTT-pyr13-Fv3C/Bgl3 fusion.
Figure 3: A map of pENTR-TOPO-Bgl1 (943/942).
Figure 4: A map of TOPO Blunt/Pegl1-Fv43D.
Figure 5: SDS gel electrophoresis analysis of Fv43D and (Fv3C/Te3A/Bgl3 chimera) expression.
Figure 6: HPLC chromatogram of Fv43D and (Fv3C/Te3A/Bgl3 chimera) produced by co-fermenting two *T. reesei* strains.
Figure 7: SDS gel electrophoresis analysis of cellulase expression by *T. reesei* RL-P37 and beta-glusosidase 1 (Bgl1, or Tr3A) expression by *T. reesei* RL-P37-d/Tr3A.
Figure 8: HPLC chromatogram of Bgl1 (Tr3A) and cellulase produced by co-fermenting *T. reesei* RL-P37 and *T. reesei* RL-P37-d/Tr3A.

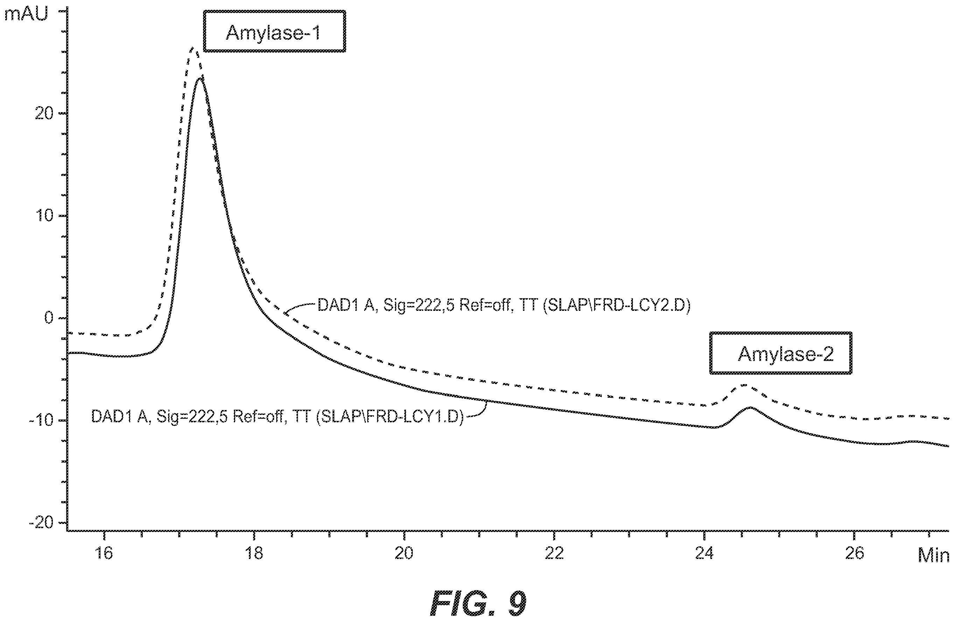

Figure 9: HPLC chromatogram of amylase variants 1 and 2 produced by co-fermenting two *B. licheniformis* strains.

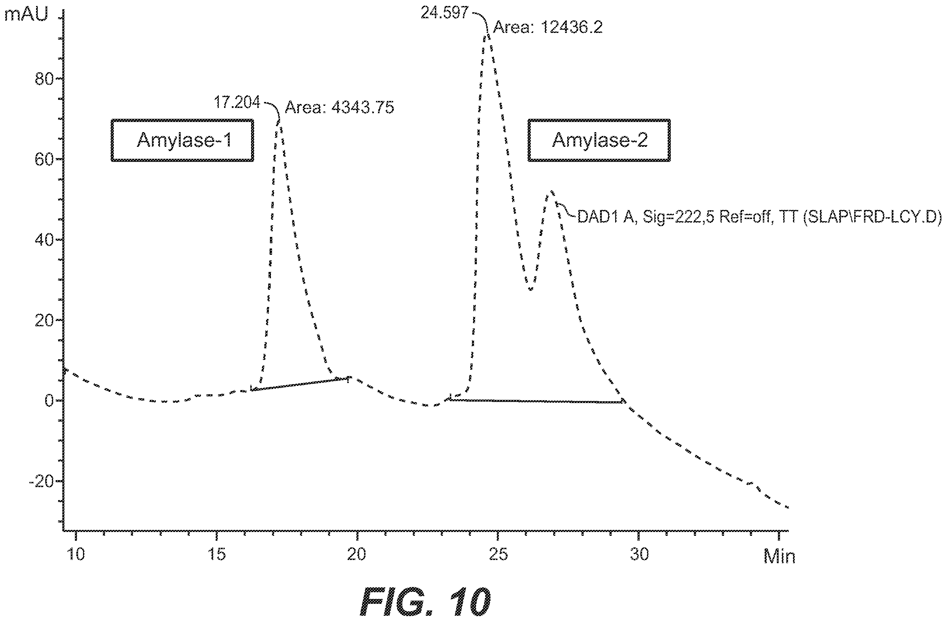

Figure 10: HPLC chromatogram of amylase variants 1 and 2 produced by co-fermenting two *B. licheniformis* strains.
Figure 11: HPLC chromatogram of (Fv3C/Te3A/Bgl3 chimera), Bgl1 (Tr3A), and glucoamylase produced by co-fermenting three *T. reesei* strains.

### SUMMARY

The invention provides methods of producing a mixture of enzymes catalyzing a conversion process, as defined in Claim 1. Such methods comprise combining first and second cell lines in a liquid medium and culturing the combined cell lines, as set forth in the claims. The cell lines secrete the enzymes into the medium, or are lysed releasing the enzymes, thereby providing a mixture of enzymes in proportions effective to enhance the conversion process. The first cell line encodes and is disposed to express a first set of one or more enzymes. The second cell line encodes and is disposed to express a second set of one or more enzymes. The first and second sets of enzymes have catalytic activities enhancing the conversion process. One or more of the first set of enzymes are heterologous to the first cell line. One or more of the second set of enzymes are heterologous to the second cell line. In some methods, one or more of the second set of enzymes are heterologous to the second cell line. In some methods, one or more of the second set of enzymes are expressed at a lower level by the first cell line. Certain methods may also include a third, a fourth, or even a fifth or sixth cell lines, each of which may secrete the enzymes into the medium or are lysed releasing the enzymes, encodes and is disposed to express a third, fourth, fifth, or sixth, respectively, set of one or more enzymes. These additional sets of enzymes also have catalytic activities enhancing the conversion process, wherein one or more of these enzymes are exogenous to the respective cell lines. One or more enzymes of these additional sets of enzymes can be exogenous to one or more of the cell lines other than the one expressing the enzymes.

Some methods further comprise identifying a plurality of enzymes catalyzing the conversion process, and identifying or constructing (1) a first cell line encoding and disposed to express a first set of one or more of the identified enzymes, and (2) a second cell line encoding and disposed to express a second set of the one or more enzymes to provide the first and second cell lines. The cell lines have been grown under selective pressure or under conditions that allow auxotroph growth to retain their disposure to expressing the enzymes to form a bank of cell lines; wherein the plurality of cell lines comprise the first cell line encoding and disposed to express a first set of one or more enzymes, and the second cell line encoding and disposed to express a second set of one or more enzymes, selecting the first and second cell lines from the bank.

Some methods of the invention can further comprise a third, a fourth, a fifth or even a sixth cell lines. The plurality of cell lines comprise the first cell line encoding and disposed to express a first set of one or more enzymes as defined in claim 1, and the second cell line encoding and disposed to express a second set of one or more enzymes as defined in claim 1. In cases where more than two cell lines are contemplated, the third, fourth, fifth or sixth cell lines encoding and disposed to express a third, fourth, fifth and sixth set of one or more enzymes. The phenotypes of each of the plurality of cell lines can be maintained by being grown under selective pressure or under conditions that allow auxotroph growth. In some methods, the cell lines are grown under different selective pressures in the identifying step and without selective pressure in the culturing step.

The invention further provides methods of preparing a cell bank as defined in claim 6.

In some methods, the first and second cell lines, or one or more other cell lines, are the same cell line engineered to encode the first and second sets, or one or more other sets, of one or more enzymes respectively.. In some embodiments, the third, fourth, fifth, or sixth set of one or more enzymes are endogenously expressed by the respective cell lines. In some methods, at least one of the first set of one or more enzyme is not encoded by the second cell line and at least one of the second set of one or more enzyme is not encoded by the first cell line. In some methods, at least one of any set of one or more enzymes is not encoded by at least one of the cell lines other than the one encoding that enzyme. In some methods, at least one enzyme of the second set is encoded and disposed to be expressed by the first cell line. In some methods, the combining step comprises combining the first, second and third cell lines, the third cell line encoding and disposed to express a third set of one or more enzymes. In some methods, the combining step comprises combining the first, second, third, fourth, fifth or sixth cell lines, wherein the latter (i.e., after the first and second) cell lines encoding and disposed to express the third, fourth, fifth and sixth set of one or more enzymes. In some methods, the first set of enzymes comprises two or more different enzymes, each having an activity enhancing the conversion process. In some methods, each set of enzymes comprises two or more different enzymes, each having an activity enhancing the conversion process.

Some methods further comprise separating the mixture of enzymes from cells in the culture. Some methods further comprise combining the mixture of enzymes with a substrate, wherein the mixture of enzymes enhances conversion of the substrate to a product. In some methods, the substrate is cellulose and/or hemicellulose and the product is glucose. In some methods, the substrate is starch and the product is sugar.

Some methods further comprise determining the proportions of the enzymes in the mixture. In some methods, the molar ratio of an enzyme of the first set of enzymes and an enzyme of the second set of enzymes in the mixture is at least two-fold different than a molar ratio of the enzymes expressed by either the first or second cell line alone. As defined in claim 1, the enzyme of the second set of enzymes is heterologous to the second cell line. In some methods, the molar ratio of the first set of enzymes that are secreted and the second set of enzymes that are secreted is at least two-fold different than a molar ratio of the enzymes expressed by either the first or second cell line alone. In some methods, the molar ratio of the first set of enzymes that are heterologous to the first cell line and the second set of enzymes that are heterologous to the second cell line is at least two-fold different than a molar ratio of the enzymes expressed by either the first or second cell line alone. In some methods, the molar ratio of the most highly expressed enzyme of the first set and the most highly expressed enzyme of the second set in the mixture is at least two-fold different than a molar ratio of the enzymes expressed by either the first or second cell line alone. In some methods, the molar ratio is at least five-fold different. In some methods, the molar ratio ranges from 1:20 to 20:1. In some methods, the molar ratio ranges from 1:5 to 5:1. In some methods, the molar ratio ranges from 1:2 to 2:1. In some embodiments, the molar ratio of an enzyme of each set of enzymes can be determined by culturing each cell line, and combining the sets of enzymes to form a mixture having the desired proportion of each enzyme.

In some methods, the first and second cell lines are the same strain. In some methods, two or more of the plurality of cell lines can be the same strain. As defined in claim 1, the first and second cell lines are the same strain modified to express different sets of one or more heterologous enzymes. In some methods, two or more of the plurality of the cell lines, which are the same strain, can be modified to express different sets of one or more heterologousenzymes. In some methods, the first and second cell lines are microbial cell lines. In some methods, one or more cell lines of the plurality of cell lines are microbial cell lines. In some methods, the first and second cell lines are the cell lines of filamentous fungal cell lines. In some methods, one or more cell lines of the plurality of cell lines are filamentous fungal cell lines. As defined in the claims, the first and second cell lines are filamentous fungal cell lines or bacterial cell lines. In some methods, one or more, or two or more cell lines of the plurality of cell lines are filamentous fungal cell lines or bacterial cell lines. In some methods, the first and second cell lines are from filamentous fungal cell lines of the same genera. In some methods, two or more cell lines of the plurality of cell lines are filamentous fungal cell lines of the same genera. In some methods, the first and second cell lines are filamentous fungal cell lines of different genera. In some methods, two or more of the plurality of cell lines are filamentous fungal cell lines of different genera. In some methods, the first and second cell lines are filamentous fungal cell lines of different species of the same genera. In some methods, two or more cell lines of the plurality of cell lines are filamentous fungal cell lines of different species of the same genera. In some methods, the first and second cell lines are filamentous fungal cell lines of different strains of the same species. In some methods, two or more cell lines of the plurality of cell lines are filamentous fungal cell lines of different strains of the same species. In some methods, the first and second cell lines are filamentous fungal cell lines of different species of the same genera. In some methods, two or more cell lines of the plurality of cell lines are filamentous fungal cell lines of different species of the same genera. In some methods, the first line is a filamentous fungal cell line and the second cell line is a bacterial cell line. In some methods, at least one of the plurality of cell lines is a filamentous fungal cell line and at least one of the plurality of cell lines is a bacterial cell line. In some methods, the first and/or second cell lines are filamentous fungal cell lines. In some methods, one or more cell lines of the plurality of cell lines are filamentous fungal cell lines. In some methods, the first and/or second cell lines are *Trichoderma reesei* cell lines. In some methods, one or more cell lines of the plurality of cell lines are *Trichoderma reesei* cell lines. In some methods, the first and/or second cell lines are bacterial cell lines. In some methods, one or more cell lines of the plurality of cell lines are bacterial cell lines. In some methods, the first and/or second cell lines are *Bacillus* cell lines. In some methods, one or more cell lines of the plurality of cell lines are *Bacillus* cell lines.

Some methods further comprise determining growth profiles of the cell lines before the combining step, e.g., determining a ratio with which to mix the cell lines based on the growth profiles. In some methods, the growth profiles of the cell lines are determined in different liquid media. Some methods further comprise selecting the liquid medium for culturing the combined cell lines based on the growth profiles of the cell lines in the different liquid media. In some methods, the growth profiles of the cell lines are determined in the same liquid media. Some methods further comprise selecting the liquid medium for culturing the combined cell lines based on the growth profiles of the cell lines in the same liquid media. In some methods, the growth rates of the cell lines are within a factor of two of one another in the selected liquid medium.

### DEFINITIONS

The cells used in the present methods can be from any type of organism, provided that the methods areas defined in the claims. Different organisms can be classified by domain (e.g., eukaryotes and prokaryotes). Domains are subdivided into kingdoms, e.g., Bacteria (e. g., Eubacteria); Archaebacteria; Protista; Fungi; Plantae; and Animalia. Kingdoms are further divided into phylums, classes, subclasses, orders, families, and genera. For example, genera from fungi include *Trichoderma, Aspergillus, Dermatophytes, Fusarium, Penicillum,* and *Saccharomyces.* Genera are further divided into species. For example, species from *Trichoderma* include *Trichoderma reesei, Trichoderma viride, Trichoderma harzianum,* and *Trichoderma koningii.* Species are divided into strains.

Different strains are independent isolates of the same species. Different strains have different genotypes and/or phenotypes.

A cell line is used in the conventional sense to indicate a population of substantially isogenic cells capable of continuous (preferably indefinite) growth and division in vitro without change other than occasional random mutations inherent from DNA replication. A cell line is typically propagated from a single colony.

Submerged fermentation is a process in which the cells grow at least predominantly under the surface of the liquid medium.

Solid state fermentation is a process in which cells grow on and inside a solid medium.

An exogenous enzyme means an enzyme that is not normally expressed by a cell (e.g., a heterologous enzyme from another strain, species, genera or kingdom) or an enzyme that is normally expressed by a cell but is expressed at an increased level by virtue of being under the control of genetic material not normally present in a cell. Such expression can result from introduction of a gene encoding such an enzyme at a location where it is not normally present or by genetic manipulation of the cell to enhance the expression of an enzyme. Such genetic manipulation can change a regulatory element controlling expression of the enzyme or can introduce genetic material encoding a protein that acts *in trans* to enhance expression of the enzyme.

An exogenous nucleic acid (e.g., DNA) means a nucleic acid not normally present in a cell (i.e., introduced by genetic engineering). An exogenous nucleic acid can be from a different strain, species, genera or kingdom (i.e., heterologous) or can be normally present in a cell but introduced in a different location than normally present.

An enzyme is endogenous to a cell if the enzyme is normally expressed by the cell, and neither nucleic acid encoding the enzyme or any other nucleic acid regulating expression of the enzyme has been introduced into cell. An endogenous gene means a gene normally present in a cell at its normal genomic location. An enzyme or nucleic acid encoding the enzyme are heterologous to a cell if not normally encoded by the cell and introduced into the cell by genetic engineering.

The term "filamentous fungi" refers to all filamentous forms of the subdivision *Eumycotina* (see, Alexopoulos, C. J. (1962), Introductory Mycology, Wiley, New York). These fungi are characterized by a vegetative mycelium with a cell wall composed of chitin, cellulose, and other complex polysaccharides. The filamentous fungi are morphologically, physiologically, and genetically distinct from yeasts. Vegetative growth by filamentous fungi is by hyphal elongation and carbon catabolism is obligatory aerobic.

A cell is disposed to express an enzyme if the cell includes DNA encoding the enzyme operably linked to one or more regulatory elements that allow expression of the DNA. The enzyme can be as defined in claim 1. Expression can be constitutive or inducible. The DNA encoding the enzyme can be in a genomic or episomal location within the cell. When two enzymes are said to be expressed at different levels by different cell lines, the ranges represented by the standard error of the mean (SEM) for the respective expression levels at the protein level do not overlap. Expression levels are compared between respective cultures of the same density and stage of culture growth of the respective cell lines. Expression levels are preferably determined from the concentration of secreted protein in culture media. Expression levels can be determined in units of moles, activity units, OD or other units.

### DETAILED DESCRIPTION

### I. Introduction

The invention provides methods of preparing enzyme cocktails by co-culturing different cell lines expressing different sets of enzymes catalyzing the same conversion process. Co-culturing provides greater flexibility and lower costs than conventional methods. It allows various mixtures to be made as needed without having to build a new production strain for each individual type of substrates and pretreatment methods. It also allows the desired enzyme mixtures to be created in one batch, obviating the need for blending the output from several separate fermentations. Preparation of a mixture of enzymes according to the present methods does not require a full recovery process for each fermentation, and/or separate storage of each enzyme component. Further, it allows maintenance of each production strain separately thereby preventing loss of the entire cocktail (engineered into a single production cell line) all at once.

### II. Conversion Process

A conversion process is a process in which a substrate is converted into a product catalyzable by at least two enzymes. The substrate can be a complex substance such as plant material containing multiple types of molecules. The product can be a single product or multiple products. The conversion process can be a single step process or involves multiple steps. The process can involve multiple sequential and/or parallel steps. Different enzymes can act in sequential steps, parallel steps or in combination on the same step. Exemplary conversion processes include the conversion of cellulosic biomass, glycogen, starch and various forms thereof into sugars (e.g., glucose, xylose, maltose) and/or alcohols (e.g., methanol, ethanol, propanol, butanol).

Some conversion processes convert starch, e.g., corn starch, wheat starch, or barley starch, corn solids, wheat solids, and starches from grains and tubers (e.g., sweet potato, potato, rice and cassava starch) into ethanol, or a syrup rich in saccharides useful for fermentation, particularly maltotriose, glucose, and/or maltose, or simply into one or more forms of sugars, which are in themselves useful products.

Some conversion processes act on cellulosic or lignocellulosic material such as materials comprising cellulose and/or hemicellulose, and sometimes lignin, starch, oligosaccharides, and/or monosaccharides. Cellulosic or lignocellulosic material can optionally further comprise additional components, such as proteins and/or lipids. Cellulosic or lignocellulosic material includes bioenergy crops, agricultural residues, municipal solid waste, industrial solid waste, sludge from paper manufacture, yard waste, wood and forestry waste, such as corn cobs, crop residues such as corn husks, corn stover, grasses, wheat, wheat straw, barley straw, hay, rice straw, switchgrass, wasted paper, sugar cane bagasse, sorghum, giant reed, elephant grass, miscanthus, Japanese cedar, components obtained from milling of grains, tress, branches, roots, leaves, wood chips, sawdust, shrubs and bushes, vegetables, fruits, flowers and animal manure. Cellulosic or lignocellulosic material can be derived from a single source, or can comprise a mixture derived from more than one source. For example, cellulosic or lignocellulosic material can comprise a mixture of corn cobs and corn stover, or a mixture of grass and leaves. Exemplary products of enzymatic conversion of the cellulosic or lignocellulosic material substrate are glucose and ethanol.

In other conversion processes, the substrate is glucose, fructose, dextrose, and sucrose, and/or C5 sugars such as xylose and arabinose, and mixtures thereof. Sucrose can be derived from sources such as sugar cane, sugar beets, cassava, sweet sorghum, and mixtures thereof. Glucose and dextrose can be derived from renewable grain sources through saccharification of starch based feedstocks including grains such as corn, wheat, rye, barley, oats, and mixtures thereof. Fermentable sugars can also be derived from cellulosic or lignocellulosic biomass through processes of pretreatment and saccharification. The product of such conversion processes can be alcohols such as ethanol or butanol.

In some conversion processes, the substrates are pretreated. Pretreatments can be mechanical, chemical, or biochemical processes or combinations thereof. The pretreatment can comprise one or more techniques including autohydrolysis, steam explosion, grinding, chopping, ball milling, compression mulling, radiation, flow-through liquid hot water treatment, dilute acid treatment, concentrated acid treatment, peracetic acid treatment, supercritical carbon dioxide treatment, alkali treatment, organic solvent treatment, , and treatment with a microorganism, such as, for example a fungus or a bacterium. The alkali treatment can include sodium hydroxide treatment, lime treatment, wet oxidation, ammonia treatment, and oxidative alkali treatment. The pretreating can involve removing or altering lignin, removing hemicellulose, decrystallizing cellulose, removing acetyl groups from hemicellulose, reducing the degree of polymerization of cellulose, increasing the pore volume of lignocellulose biomass, increasing the surface area of lignocellulose, or any combination thereof.

### III. Enzymes

Cocktails of any combination of enzymes selected from enzymes including, but not limited to, the six major enzyme classifications of hydrolase, oxidoreductase, transferase, lyase, isomerase or ligase can be made (Nomenclature Committee of the International Union of Biochemistry and Molecular Biology (NC-IUBMB), Enzyme Nomenclature, Academic Press, San Diego, California, 1992). Examples of suitable enzymes include a cellulase, a hemicellulase, a xylanase, an amylase, a glucoamylase, a protease, a phytase, a cutinase, a phytase, a laccase, a lipase, an isomerase, a glucose isomerase, an esterase, a peroxidase, a phospholipase, a pectinase, a keratinase, a reductase, an oxidase, a peroxidase, a phenol oxidase, a lipoxygenase, a ligninase, a pullulanase, a tannase, a pentosanase, a maltase, mannanase, glucuronidase, galactanase,, a β-glucanase, an arabinosidase, a hyaluronidase, a lactase, a polygalacturonase, a β-galactosidase, and a chondroitinase, or any enzyme for which closely related and less stable homologs exist.

The enzymes can be from any origin, e.g., bacteria or fungi. The enzymes can be a hybrid enzyme, i.e., a fusion protein which is a functional enzyme, wherein at least one part or portion is from a first species and another part or portion is from a second species. The enzymes can be a mutant, truncated or hybrid form of native enzymes. The enzymes suitable for the present methods can be a secreted, cytoplasmic, nuclear, or membrane protein. Extracellular enzymes, e.g., a cellulase, hemicellulase, protease, or starch degrading enzyme such as amylase, usually have a signal sequence linked to the N-terminal portion of their coding sequence.

Examples of enzyme substrates include lignocellulosic materials, cellulose, hemicellulose, starch, or a combination thereof. An exemplary group of enzymes for catalyzing lignocellulosic materials conversion includes endoglucanases, exoglucanases or cellobiohydrolases and β-glucosidases. An exemplary group of enzymes for catalyzing hemicellulose conversion includes at least xylanase, mannanase, xylosidase, mannosidase, glucosidase, arabinosidase, glucuronidase, and galactosidase. An exemplary group of enzymes for catalyzing starch hydrolysis include at least α-amylase, saccharifying α-amylase, β-amylase, glucoamylase, and pullulanases. Depending on the raw materials and pre-treatment methods, additional enzymes, e.g., proteases and phytases, can be selected.

Cellulases are enzymes that hydrolyze the β-D-glucosidic linkages in celluloses. Cellulolytic enzymes have been traditionally divided into three major classes: endoglucanases, exoglucanases or cellobiohydrolases and β-glucosidases (Knowles, J. et al., TIBTECH 5:255-261 (1987)). Cellulase enzymes also include accessory enzymes, including GH61 members, such as EG4, swollenin, expansin, and CIP1. Numerous cellulases have been described in the scientific literature, examples of which include: from *Trichoderma reesei:* Shoemaker, S. et al., Bio/Technology, 1:691-696, 1983, which discloses CBHI; Teeri, T. et al., Gene, 51:43-52, 1987, which discloses CBHII; Penttila, M. et al., Gene, 45:253-263, 1986, which discloses EGI; Saloheimo, M. et al., Gene, 63:11-22, 1988, which discloses EGII; Okada, M. et al., Appl. Environ. Microbiol., 64:555-563, 1988, which discloses EGIII; Saloheimo, M. et al., Eur. J. Biochem., 249:584-591, 1997, which discloses EGIV; and Saloheimo, A. et al., Molecular Microbiology, 13:219-228, 1994, which discloses EGV Exo-cellobiohydrolases and endoglucanases from species other than *Trichoderma* have also been described e.g., Ooi et al., 1990, which discloses the cDNA sequence coding for endoglucanase F1-CMC produced by *Aspergillus aculeatus;* Kawaguchi T et al., 1996, which discloses the cloning and sequencing of the cDNA encoding β-glucosidase 1 from *Aspergillus aculeatus*; Sakamoto et al., 1995, which discloses the cDNA sequence encoding the endoglucanase CMCase-1 from *Aspergillus kawachii* IFO 4308; and Saarilahti et al., 1990 which discloses an endoglucanase from *Erwinia carotovara.*

Hemicellulases are enzymes that catalyze the degradation and/or modification of hemicelluloses, including xylanase, mannanase, xylosidase, mannosidase, glucosidase, arabinosidase, glucuronidase, and galactosidase. For example, the hemicellulase can be a xylanase, i.e., any xylan degrading enzyme which is either naturally or recombinantly produced. Generally, xylan degrading enzymes are endo- and exo-xylanases hydrolyzing xylan in an endo- or an exo-fashion. Exemplary xylan degrading enzymes include endo-1,3-β-xylosidase, endo-β1,4-xylanases (1,4-β-xylan xylanohydrolase; EC 3.2.1.8), 1,3-β-D-xylan xylohydrolase and β-1-4- xylosidases (1,4-β-xylan xylohydrolase; EC 3.2.1.37) (EC Nos. 3.2.1.32, 3.2.1.72, 3.2.1.8, 3.2.1.37). Preferred xylanases are those which are derived from a filamentous fungus (e.g., the fungi of the genera *Aspergillus, Disportrichum, Penicillium, Humicola, Neurospora, Fusarium, Trichoderma* and *Gliocladium*) or a bacterial source (e.g., *Bacillus, thermotoga, Streptomyces, Microtetraspora, Actinmadura, Thermomonospora, Actinomyctes* and *Cepholosporum*)*.*

Amylases are starch-degrading enzymes, classified as hydrolases, which cleave α-D-(1→4) O-glycosidic linkages in starch. Generally, α-amylases (E.C. 3.2.1.1, α-D-(1→4)-glucan glucanohydrolase) are defined as endo-acting enzymes cleaving α-D-(1→4) O-glycosidic linkages within the starch molecule in a random fashion. The exo-acting amylolytic enzymes, such as β-amylases (E.C. 3.2.1.2, α-D-(1→4)-glucan maltohydrolase), and some product-specific amylases like maltogenic alpha-amylase (E.C. 3.2.1.133) cleave the starch molecule from the non-reducing end of the substrate. β-Amylases, α-glucosidases (E.C. 3.2.1.20, α-D-glucoside glucohydrolase), glucoamylase (E.C. 3.2.1.3, α-D-(1→4)-glucan glucohydrolase), and product-specific amylases can produce malto-oligosaccharides of a specific length from starch.

Preferably, α-amylases are those derived from *Bacillus* sp., particularly those from *Bacillus licheniformis, Bacillus amyloliquefaciens* or *Bacillus stearothermophilus,* as well as *Geobacillus stearothermophilus,* and fungal α-amylases such as those derived from *Aspergillus* (i.e., A. *oryzae* and A. *niger*). Optionally, α-amylases can be derived from a precursor α-amylase. The precursor α-amylase is produced by any source capable of producing α-amylase. Suitable sources of α-amylases are prokaryotic or eukaryotic organisms, including fungi, bacteria, plants or animals. Preferably, the precursor α-amylase is produced by *Geobacillus stearothermophilus* or a *Bacillus*; more preferably, by *Bacillus licheniformis, Bacillus amyloliquefaciens* or *Bacillus stearothermophilus;* most preferably, the precursor α-amylase is derived from *Bacillus licheniformis.* α-amylases can also be from *Bacillus subtilis.*

Glucoamylases are enzymes of amyloglucosidase class (E.C. 3.2.1.3, glucoamylase, 1,4-alpha-D-glucan glucohydrolase). These enzymes release glucosyl residues from the non-reducing ends of amylose and amylopectin molecules.

Pullulanases are starch debranching enzymes. Pullulanases are enzymes classified in EC 3.2.1.41 and such enzymes are characterized by their ability to hydrolyze the α-1, 6-glycosidic bonds in, for example, amylopectin and pullulan.

Other enzymes include proteases, such as a serine, metallo, thiol or acid protease. Serine proteases (e.g., subtilisin) are described by e.g., Honne-Seyler's Z Physiol. Chem 364:1537-1540, 1983; Drenth, J. et al. Eur. J. Biochem. 26:177-181, 1972; U.S. Pat. Nos. 4,760,025 (RE 34,606), 5,182,204 and 6,312,936 and EP 0 323,299). Proteolytic activity can be measured as disclosed in K. M. Kalisz, "Microbial Proteinases" Advances in Biochemical Engineering and Biotechnology, A. Fiecht Ed. 1988.

Phytases are enzymes that catalyze the hydrolysis of phytate to (1) myo-inositol and/or (2) mono-, di-, tri-, tetra- and/or penta-phosphates thereof and (3) inorganic phosphate. For example, phytases include enzymes defined by EC number 3.1.3.8, or EC number 3.1.3.26.

### IV. Cell lines

Having selected a conversion process and identified from published literature and/or by experimentation one or more combinations of enzymes expected to enhance the conversion process, cell lines are identified or constructed to express different sets of the enzymes. The enzymes endogenously expressed by some cell lines are well known. For example, *T. reesei* is a source of several cellulose processing enzymes and *Bacillus* is a source of a number of amylases. Such cell lines are sometimes used without modification. Often, however, one or more enzymes desired to enhance the enzymatic conversion process are not endogenously expressed at sufficient levels by a known existing cell line. In this case, an existing cell line can be genetically engineered to express an enzyme exogenously. If several enzymes desired to enhance the conversion process are not expressed at sufficient levels by a known existing cell line, existing cell line(s) can be genetically engineered to express each of the enzymes exogenously. For maximum modularity, each such enzyme can be exogenously expressed in its own cell line. Preferably, the cell lines into which different enzymes are genetically engineered represent modifications of the same base cell line.

As a result of endogenous expression, exogenous expression, or both, cell lines to be co-cultured can express different sets or panels of enzymes, all of which contribute to the enhancement of enzymatic conversion. For a cell line that does not express any endogenous enzyme enhancing the conversion process, and which has been genetically engineered to express one or more exogenous enzymes, the set or panel of enzymes produced by the cell line are said to include exogenous enzyme(s). In a cell line that endogenously expresses enzyme(s) enhancing the conversion process, and which has been genetically engineered to express one or more exogenous enzymes, the set or panel of enzymes produced by the cell line are said to include endogenous enzymes and exogenous enzymes. In a cell line that has not been genetically engineered to express an exogenous enzyme, the set or panel of enzymes produced by the cell line are said to include only endogenous enzymes. Although exogenous enzymes of a set are readily known and recognized, such is not necessarily the case for endogenous enzymes expressed at trace levels. For this reason, the set or panel of enzymes is defined as including only enzymes expressed at detectable levels as determinable by HPLC according to the conditions and/or protocols used in the examples. Preferably each enzyme in a set is expressed and/or secreted at a level of at least 1/100 or 1/10 the level of the most highly expressed enzyme in the set. It is not necessary for practice of the present methods to know the identity of all enzymes falling within a set. Rather, it is sufficient to know the identity of at least one enzyme within a set produced by a given cell line.

The set of enzymes encoded by one cell line can contain no, partial or complete overlap with the set of enzymes encoded by a second cell line. Enzymes present in the first set of the first cell line and enzymes present in the second set of the second cell line may be expressed at different levels. If the identities of the enzymes in the sets completely overlap at least one enzyme is expressed at a different level (i.e., the standard errors of means (SEMs) do not overlap) between the sets. Preferably, each set of enzymes includes at least one enzyme not expressed or expressed at a lower level in other set(s) of enzymes from other cell line(s) included in the co-culture. Preferably at least one enzyme in one set of enzymes (e.g., a first set) catalyzing a conversion process is heterologous to that cell line expressing the same set of enzymes (e.g., a first cell line). Preferably any cell line included in a co-culture not expressing an exogenous enzyme expresses an endogenous enzyme, which is otherwise not expressed or expressed at significantly lower levels by each other cell line included in the co-culture. When one set of enzymes is as defined in claim 1, the cell lines expressing the other sets can be a strain, a species, or a genus different than that of the first cell line. Alternatively, one cell line can be a base strain or cell line modified to express an exogenous enzyme and another cell line can the base cell line or strain without the modification. Although it might be thought that co-expression of the modified cell line with the base cell line would undesirably dilute the relative concentration of exogenous enzyme relative to endogenous enzymes produced by the base cell line, in fact, the modification may substantially suppress expression of an endogenous enzyme that would otherwise enhance the conversion process. In this situation, co-cultivation of the modified cell line with the base strain or cell line can provide a blend of the exogenous and endogenous enzymes in more effective proportions than culture of either cell line alone.

By co-culturing two or more cell lines, different sets of enzymes can be expressed together, achieving ratios of enzymes or enzymatic activities different than those of each cell line alone. The ratios are preferably by moles but activity units, mass or other units can also be used.

The ratio of any enzymes can be compared by assessing the difference between 1) a first set of enzymes and a second set of enzymes in a mixture of enzymes resulting from co-culture and 2) one or both individual cell lines. Such a comparison is most readily illustrated on a pair-wise basis between the most highly expressed enzyme in the first set and the most highly expressed enzyme in the second set (expression being measured at the protein level, preferably of a secreted protein). The ratio of such enzymes in either individual cell line is preferably at least 2, 3, 4, 5, 10, 15, 20, 25, 30, 35, 40, 45, or 50-fold different than in the mixture of enzymes. For example, if the highest expressed enzyme in a first set and the highest expressed enzyme in a second set are expressed at a 1:1 molar ratio in a mixture resulting from co-culture and a 10:1 ratio in a first cell line and a 1:10 ratio in a second cell line, then the molar ratio is 10-fold different in the mixture than either cell line. Pair-wise or group comparisons can be made between any other enzymes in the first or second set. A group used for a comparison can be defined as, e.g., secreted enzymes in each set, intracellular enzymes in each set, exogenous enzymes in each set, or enzymes having a recombinant tag in each set.

The ratio of enzymes between the first and second sets can also be compared by summing the molar amounts of known enzymes in the first set and molar amounts of enzymes in the second set (or at least those that are known) and calculating a ratio. Such ratios preferably range from 1:50 to 50:1, 1:45 to 45:1, 1:40 to 40:1, 1:35 to 35:1, 1:30 to 30:1, 1:25 to 25:1, 1:20 to 20:1, 1:10 to 10:1, 1:5 to 5:1, or 1:2 to 2:1

As disclosed herein, cell lines may be engineered to express one or more exogenous enzymes by conventional methods. In some such methods, a nucleic acid encoding an enzyme in operable linkage to regulatory sequences to ensure its expression is transformed into the cell line. Optionally the enzyme can be fused to a recombinant tag (e.g., His-tag, FLAG-tag, GST, HA-tag, MBP, Myc-tag) to facilitate detection or quantification in co-culture or in a mixture of enzymes resulting from co-culture. The nucleic acid encoding the enzyme is preferably also fused to a signal peptide to allow secretion. Any suitable signal peptide can be used depending on the enzyme to be expressed and secreted in a host organism. Examples of signal sequences include a signal sequence from a *Streptomyces* cellulase gene. A preferred signal sequence is a *S. lividans* cellulase, celA (Bently et al., Nature 417:141-147, 2002). The nucleic acid is then preferably stably maintained either as a result of transformation on an episome or through integration into the chromosome. Alternatively, expression of an enzyme can be induced by activating in *cis* or in *trans* DNA encoding the enzyme in the chromosome.

As well as engineering cell lines to express an exogenous gene, it is sometimes desirable to engineer cell lines to inhibit or knockout expression of an endogenous gene encoding a product that is an inhibitor to the conversion process. The inhibition or knockout strategy can also be used to remove unnecessary genes or replacing an endogenous gene and replacing it with an improved version, a variant of, and/or a heterologous version of that gene. Such inhibition or knockout can be performed by siRNA, zinc finger proteins, other known molecular biology techniques used to knockout or reduce expression of particular endogenous genes, or the like.

The cell lines combined for co-culture can be from different, or same, domains, kingdoms, phylums, classes, subclasses, orders, families, genera, or species. They can also be from different strains of different species, different strains of the same species, or from the same strain.

Exemplary combinations include cell lines from different strains of the same species (e.g., *T. reesei* RL-P37 (Sheir-Neiss and Montenecourt, Appl. Microbiol. Biotechnol. 20:46-53, 1984) and *T. reesei* QM-9414 (ATCC No. 26921; isolated by the U.S. Army Natick Laboratory). Cell lines from different strains of different species in the same kingdom (e.g., fungus) can be used (e.g., *T. reesei* RL-P37 and *Aspergillus niger*). Cell lines from different strains of different species in different kingdoms/domains can also be used (e.g., bacteria, yeast, fungi, algae, and higher eukaryotic cells (plant or animal cells)). Exemplary combinations further include a bacterium (e.g., *B. subtilis* or *E. coli*) and a fungus (e.g., *T. reesei* or *Aspergillus niger*); a bacterium and a yeast (e.g., *Saccharomyces* or *Pichia*); a yeast and a fungus; a bacterium and an algae, a yeast and an algae, a fungus and an algae and so forth.

When two or more cell lines are engineered from a same base strain (e.g., *T. reesei,* RL-P37 or *B. subtilis*), each cell line can encode one or more different enzymes as defined in claim 1. Optionally, some cell lines can also be engineered so that a gene in the base strain is suppressed or inhibited, e.g., by at least 50%, 75%, or 90%, of the normal expression level.

The cell lines suitable for the present methods include bacteria, yeast, fungi and higher eukaryotic cell lines such as plant or animal cell lines. Microbial cell lines are preferred.

Cell lines additional to the first and second cell lines can be yeast cell lines. Examples of yeast cells include *Saccharomyces* sp., *Schizosaccharomyces* sp., *Pichia* sp., *Hansenula* sp., *Kluyveromyces* sp., *Prtaffia* sp., or *Candida* sp., such as *Saccharomyces cerevisiae, Schizosaccharomyces pombe, Candida albicans, Hansenula polymorpha, Pichia pastoris, P. canadensis, Kluyveromyces marxianus,* and *Phaffia rhodozyma.*

Cell lines additional to the first and second cell lines can be fungal cell lines. Examples of fungi include species of *Aspergillus* such as *A. oryzae* and *A. niger,* species of *Saccharomyces* such as *S. cerevisiae,* species of *Schizosaccharomyces* such as *S. pombe,* and species of *Trichoderma* such as *T. reesei.*

Preferred examples of fungi include filamentous fungal cells. The filamentous fungal parent cell may be a cell of a species of, but not limited to, *Trichoderma,* (e.g., *Trichoderma reesei,* the asexual morph of *Hypocrea jecorina,* previously classified as *T. longibrachiatum, Trichoderma viride, Trichoderma koningii, Trichoderma harzianum)* (Sheir-Neiss et al, Appl. Microbiol. Biotechnol 20: 46-53, 1984; ATCC No. 56765 and ATCC No. 26921); *Penicillium* sp., *Humicola* sp. (e.g., *H. insolens, H. lanuginose,* or *H. grisea*); *Chrysosporium* sp. (e.g., *C*. *lucknowense*), *Gliocladium* sp., *Aspergillus* sp. (e.g., *A. oryzae, A. niger, A sojae, A. japonicus, A. nidulans,* or *A*. *awamori*) (Ward et al., Appl. Microbiol. Biotechnol. 39: 7380743, 1993 and Goedegebuur et al., Genet 41: 89-98, 2002), *Fusarium* sp., (e.g., *F. roseum, F. graminum, F. cerealis, F. oxysporuim,* or *F. venenatum*), *Neurospora* sp., (e.g., *N. crassa), Hypocrea* sp., *Mucor* sp., (e.g., *M. miehei*), *Rhizopus* sp. and *Emericella* sp. (see also, Innis et al, ScL 228: 21-26, 1985). The term *"Trichoderma"* or *"Trichoderma* sp." or *"Trichoderma* spp." refers to any fungal genus previously or currently classified as *Trichoderma.* The fungus can be *A. nidulans, A. awamori, A. oryzae, A. aculeatus, A. niger, A. japonicus, T. reesei, T. viride, F. oxysporum,* or *F. solani. Aspergillus* strains are disclosed in Ward et al., Appl. Microbiol. Biotechnol. 39:738-743, 1993 and Goedegebuur et al., Curr Gene 41:89-98, 2002. Preferably, the fungus is a strain of *Trichoderma,* such as a strain of *T. reesei.* Strains of *T. reesei* are known and non-limiting examples include ATCC No. 13631, ATCC No. 26921, ATCC No. 56764, ATCC No. 56765, ATCC No. 56767, and NRRL 15709. The host strain can be a derivative of RL-P37 (Sheir-Neiss et al., Appl. Microbiol. Biotechnology 20:46-53, 1984).

The cell lines can be bacterial cell lines. Examples of bacterial cells suitable for the present methods include a gram-positive bacterium (e.g., *Streptomyces* and *Bacillus*) and a gram-negative bacterium (e.g., *Escherichia coli* and *Pseudomonas* sp.). Preferred examples include strains of *Bacillus* such as *B. lichenformis* or *B. subtilis,* strains of *Lactobacillus*, strains of *Streptococcus,* strains of *Pantoea* such as *P. citrea,* strains of *Pseudomonas* such as *P. alcaligenes,* strains of *Streptomyces* such as *S. albus, S. lividans, S. murinus, S. rubiginosus, S. coelicolor,* or *S. griseus,* or strains of *Escherichia* such as *E. coli.* The genus *"Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus*, *Anoxybacillus, Brevibacillus, Filobacillus, Gracilibacillus, Halobacillus, Paenibacillus*, *Salibacillus, Thermobacillus*, *Ureibacillus,* and *Virgibacillus.*

Cell lines additional to the first and second cell lines can be plant cell lines. Examples of plant cells include a plant cell from the family *Fabaceae,* such as the *Faboideae* subfamily. Examples of plant cells suitable for the present methods include a plant cell from *kudzu, poplar* (such as *Populus alba x tremula* CAC35696 or *Populus alba*) (Sasaki et al., FEBS Letters 579(11): 2514-2518, 2005), *aspen* (such as *Populus tremuloides*), or *Quercus robur.*

Cell lines additional to the first and second cell lines can be an algae cell, such as a green algae, red algae, *glaucophytes, chlorarachniophytes, euglenids, chromista,* or *dinoflagellates.*

The cell lines can be a cyanobacteria cell, such as cyanobacteria classified into any of the following groups based on morphology: *Chroococcales, Pleurocapsales, Oscillatoriales, Nostocales,* or *Stigonematales.*

Cell lines additional to the first and second cell lines can be a mammalian cell such as Chinese hamster ovary (CHO) cells, HeLa cells, baby hamster kidney (BHK) cells, COS cells, or any number of other immortalized cell lines available, e. g., from the American Type Culture Collection.

In some methods, the first cell line is a *T. reesei* strain encoding a heterologous β-xylosidase and the second cell line is a *T. reesei* strain encoding a heterologous β-glucosidase.

In some methods, the second cell line is a *B. licheniformis* strain encoding *Geobacillus stearothermophilus* amylase.

In some methods, for example, the first cell line is a *T. reesei* strain encoding a heterologous GH61 enzyme and the second cell line is a *T. reesei* strain encoding heterologous cellulases.

### V. Co-Culturing Methods

The cell lines to be co-cultured can in some embodiments be separately cultured initially to form starter cultures (which preferably have an optical density of at least about 0.1, 0.2, 0.4, 0.8, 1.0, or 1.5 at a wavelength of 600 nm and a path length of 1 cm). The starter cultures are then mixed in equal volumes or other desired ratio (as discussed further below) in fresh culture media to form a starting co-culture. Optionally, isolates can be directly inoculated in culture media for protein production (e.g., without the use of starter cultures).

Potential issues of one cell line outgrowing another can be reduced by selecting cell lines, e.g., closely related cell lines, with inherently similar growth characteristics, selection of a culture media that is not optimal for at least one of the cell lines but reduces differences in growth when each cell line is grown on separate culture media and/or by adjusting the ratio by volume, or more accurately by OD or cell count, with which cultures are combined in order to compensate for different growth characteristics.

One source of closely related cell lines is cell lines from the same species (e.g., *T. reesei*) or same strain, or more preferably the same base strain or cell line modified in different ways to express different exogenous enzymes. For example, a first cell line is a base cell line genetically engineered to express enzyme A and a second cell line is the base cell line genetically engineered to express enzyme B.

Before combining the cell lines for co-culturing, the growth profile of each cell line can be determined. Based on the determined growth profiles, a ratio or a range of ratios, with which to mix the cell lines for optimal co-expression of the first set of enzymes and the second set of enzymes (or more sets of enzymes), can then be determined to compensate at least in part for differences in growth profiles.

In any cell culture system, there is a characteristic growth pattern following inoculation that includes a lag phase, an accelerated growth phase, an exponential or "log" phase, a negative growth acceleration phase and a plateau or stationary phase. The log and plateau phases give information about the cell line, the population doubling time during log growth, the growth rate, and the maximum cell density achieved in plateau. For example, in the log phase, as growth continues, the cells reach their maximum rate of cell division, and numbers of cells increase in log relationship to time. By making one count at a specified time and a second count after an interval during the log phase and knowing the number of elapsed time units, one can calculate the total number of cell divisions or doublings, the growth rate and generation time.

Measurement of the population doubling time can be used to monitor the culture during serial passage and calculate cell yields and the dilution factor required at subculture. The population doubling time is an average figure and describes the net result of a wide range of cell division rates within the culture. The doubling time differs with varying cell types, culture vessels and conditions. Pre-determined growth profiles can be used to determine the population doubling time for each cell line used in the co-culture. Preferably, the population doubling times in exponential growth of cell lines to be co-cultured are within a factor of 2 or 5 of each other. For example, the population doubling time in exponential growth of cell lines selected to be co-cultured are within a factor of 2, 3, 4, or 5 of each other. If the growth rates differ more broadly, then the culture media is preferably varied to identify a culture media on which the population doubling times are more similar, preferably within a factor of 2 or 5 of each other. For example, the components and conditions provided by the culture media can be adjusted and used to reduce the differences in population doubling time in exponential growth of cell lines such that the population doubling times for each cell lines become within a factor of 2 or 5 of each other. Additionally, cell lines can first be selected based on their small differences in growth profiles using conventional culture media, followed by adjustment of culture media/conditions such that the growth profiles differences become even smaller.

The optimal ratio of sets of enzymes encoded by a first cell line to a second cell line is not necessarily known *a priori.* Combination of the cell lines in different ratios by volume, OD or number of cells allows different ratios to be compared empirically on a small scale, with an optimal ratio identified by such analysis being used for subsequent larger scale culture.

To ensure no single cell line unacceptably outcompetes one or more other cell lines, e.g., by growing more rapidly and suppressing the growth of other cell lines, the cell lines can be at ratios that result in each cell line reaching a defined point in the growth curve at about the same time. For example, the ratio can be adjusted so each cell line reaches mid-log phase at about the same time. Alternatively, each cell line reaches plateau phase (mid-plateau phase) at about the same time. Preferably, each cell line reaches both the mid-log phase and the plateau phase at about the same time. Optionally, each cell line reaches stationary phase at about the same time.

The growth profiles can also be used to determine the harvest time and/or seeding densities required for achieving certain ratios of harvesting cell densities between/among the cell lines. For example, an equal molar ratio of different sets of enzymes may be desired for one type of substrates/pretreatment methods. Different ratios of enzymes desired for other types of substrates/pretreatment methods can be achieved by varying the seeding densities of one or more cell lines as well as the harvest time.

Each cell line can have different requirement for optimal growth in culture media, particularly for cell lines from different organisms (e.g., different domains, kingdoms, genera, or species), or different strains. However, a culture media, although not optimal for any single cell line, can be optimal for co-fermentation of all cell lines if all cell lines have similar growth profiles in such a media. Accordingly, the growth profile of each cell line in multiple culture media can be determined. These growth profiles are then compared to identify a culture media in which the growth profiles of the cell lines are the most similar. For example, in such a media each cell line reaches plateau phase (mid-plateau phase), mid-log phase, and/or stationary phase at about the same time. The chosen culture media is then used for co-culture.

As an alternative to, or in combination with, the cell density-based growth profiling, the amounts of the enzymes and/or the activities of the expressed enzymes can be measured along the growth curve. These variations along the growth curve provide guidance for determining the ratio with which to mix the cell lines for optimal co-expression of the enzymes. For example, the expression levels of some enzymes may be lower than other enzymes. For these enzymes, a higher seeding density of the cell lines expressing the enzymes is preferred to achieve a desired amount of these lowly expressed enzymes.

Cell lines from the same strain usually have similar growth profiles and require similar culture media. On the other hand, cell lines from different strains or different organisms often have different growth profiles and require different culture media. As discussed above, growth profiles of different cell lines can be measured to determine the seeding density for each cell line. Optionally, growth profiles in various culture media for each cell line are measured to determine a media suitable for co-culture.

The enzymes can be released directly to the culture media. Alternatively cells can be lysed releasing intracellular enzymes. Furthermore, some enzymes expressed by a given cell line can be released directly whereas other enzymes may be released by cell lysis. The released enzymes, whether as a result of secretion or lysis, can be harvested from the culture media, or the culture medium can be used as is with minimal if any further processing as a whole broth. Cell debris (e.g., host cells, lysed fragments), can optionally be removed by, e.g., centrifugation or ultrafiltration if desired. Optionally, the enzyme mixture can be concentrated, e.g., with a commercially available protein concentration filter. The enzyme mixture can be separated further from other impurities by one or more purification steps, e.g., immunoaffinity chromatography, ion-exchange column fractionation (e.g., on diethylaminoethyl (DEAE) or matrices containing carboxymethyl or sulfopropyl groups), chromatography on Blue-Sepharose, CM Blue-Sepharose, MONO-Q, MONO-S, lentil lectin-Sepharose, WGA-Sepharose, Con A-Sepharose, Ether Toyopearl, Butyl Toyopearl, Phenyl Toyopearl, or protein A Sepharose, SDS-PAGE chromatography, silica chromatography, chromatofocusing, reverse phase HPLC (RP-HPLC), gel filtration using, e.g., Sephadex molecular sieve or size-exclusion chromatography, chromatography on columns that selectively bind the peptide, and ethanol, pH or ammonium sulfate precipitation, membrane filtration and various techniques. In some methods, the enzyme mixture is used in downstream application with minimal, if any, further processing.

The amounts of the enzymes secreted or lysed from cells or in finished product can be measured using conventional techniques, e.g., by reverse phase high performance liquid chromatography (RP-HPLC), or sodium dodecyl sulfate-polyacrylamide gel electrophoresis (SDS-PAGE). The activities of the enzymes can also be measured using methods well-known in the art.

### VI. Cell Banking

Cell lines expressing different sets of enzymes can be stored in a cell bank and co-cultured in different combinations. A cell bank can be constructed with a particular conversion process or a particular set of conversions in mind. Enzymes enhancing the conversion process are identified either from known or published sources, from experiments, or from both. Cell lines are then identified or constructed encoding and disposed to express different sets of the enzymes. Cell lines expressing one or more of the enzymes endogenously or exogenously may already be known. Cell lines expressing one or more of the enzymes exogenously can also be constructed particularly if no cell line expressing a particular enzyme or a particular combination or panel of enzymes at sufficient levels is already available.

The cell lines in a bank can be stored on solid or liquid media in the cold or frozen. Before use, a vial of cells is typically separately propagated to form a starter culture, which can also take place in a liquid or on solid medium. The cell lines can be propagated and stored under different selective pressures to retain expression of the respective sets of enzyme and avoid the possibility of cross contamination. Alternatively, the cell lines can be propagated and stored under conditions that allow growth of the auxotrophs, thereby maintaining the genotypes.

The cell lines can be co-cultured and used to prepare a mixture of enzymes using the methods described above. After combination, the cell lines are propagated on media in which the combined cell lines are used, so selective conditions or conditions that allow auxotroph growth that may have been used for separate propagation and storage of the cells lines are not necessarily used in the co-culture step.

The cell bank may allow selection of different permutations of cell lines that provide enzymes enhancing the conversion process in different combinations or relative expression levels. The different combinations can be compared to determine which given enzyme mixture has the best activity for enhancing the conversion process. Such a comparison can indicate the best combination of cell lines within a bank without necessarily knowing *a priori* exactly which enzymes or what ratio of enzymes is optimal. In that sense, this allows the tailoring of the panel of expressed enzymes from a co-culture to the particular requirements of a particular conversion process.

Variations in the substrates or pretreatment of substrates for a different process can be accommodated by varying the ratio in which starter cultures of cell lines from the cell bank are combined. For example, the amount of hemicellulose may vary in a cellulose preparation. Enzyme cocktails for treating high amounts of hemicellulose can contain a higher level of xylanase activity. Some starch preparations may contain a substance (e.g., raw material or metabolite) known to be inhibitory of amylase activity, in which case a higher amylase amount is desirable. Depending on the compositions (e.g., different glucan/xylan profile) in the pretreated substrates, different enzyme cocktails can be prepared by mixing starter cultures of enzyme production strains in different ratios, thereby producing enzyme cocktails having different relative amounts of the enzymes.

Cell banking can also be useful irrespective of the conversion process. By banking different cell lines encoding a variety of commonly used industrial enzymes, the cell lines can be combined in different combinations from the bank for co-culture depending on the conversion process at hand. The co-fermentation method provided herein therefore not only provides flexibility of a resulting composition, but also affords various other advantages such as, for example, reduced costs as compared to conducting fermentation of each desired enzyme component separately followed by blending; reduced cost for storage of enzymes because co-fermentation results in a composition with desired ratios of enzymes, whereas the blending strategy will require storage for each individual enzyme separately fermented or prepared.

### VII. Applications

The enzyme mixtures produced by the present methods have various agricultural, industrial, medical and nutritional applications where such a conversion process is utilized. The substrates of such a conversion process can be, e.g., lignocellulosic materials, cellulose, hemicellulose, starch.

For example, a mixture of cellulase enzymes and/or cellulase accessory enzymes can be used for hydrolysis of cellulolytic materials, e.g., in the fermentation of biomass into biofuels. The mixture is also useful for generating glucose from grain, or as a supplement in animal feed to decrease the production of fecal waste by increasing the digestibility of the feed. Cellulase enzymes can also be used to increase the efficiency of alcoholic fermentations (e.g., in beer brewing) by converting lignocellulosic biomass into fermentable sugars. The cellulase mixture can be used for commercial food processing in coffee, i.e., hydrolysis of cellulose during drying of beans. They have also been used in the pulp and paper industry for various purposes. In pharmaceutical applications, cellulases are useful as a treatment for phytobezoars, a form of cellulose bezoar found in the human stomach.

A mixture of cellulase enzymes, cellulase accessory enzymes, and/or hemicellulase enzymes are widely used in textile industry and in laundry detergents. Cellulases can also be used in hydrolyzing cellulosic or lignocellulosic materials into fermentable sugars.

A mixture of amylases or a mixture of α-amylase, β-amylase, glucoamylase, and/or pullulanases has various applications in food industry. For example, a mixture of amylase enzymes is useful in syrup manufacture, dextrose manufacture, baking, saccharification of fermented mashes, food dextrin and sugar product manufacture, dry breakfast food manufacture, chocolate syrups manufacture, and starch removal from fruit juices. Amylases can also be used in producing glucose from grain products for ethanol production.

A mixture of enzymes containing phytases can be used in grain wet milling and cleaning products. They also find many other uses in personal care products, medical products and food and nutritional products, as well as in various industrial applications, particularly in the cleaning, textile, lithographic and chemical arts.

### EXAMPLES

**Example 1:** Co-fermentation of two *T. reesei* strains for producing a mixture of β-xylosidase and β-glucosidase.

### Protein Expression Analyzed by SDS-PAGE:

NuPAGE® Novex 4-12% Bis-Tris gels and MOPS (Invitrogen) buffer were used with the SeeBlue®Plus2 molecular weight marker for SDS-PAGE analysis. Samples were added on an equal volume of culture supernatant basis.

### Protein Expression Analyzed by HPLC:

Liquid chromatography (LC) and mass spectroscopy (MS) were performed to separate and quantify the enzymes contained in fermentation broths. In some cases, enzyme samples were treated with a recombinantly expressed endoH glycosidase from *S. plicatus* (*e.g.,* NEB P0702L) before HPLC analysis. EndoH was used at an amount of 0.01-0.03 µg endoH per µg of total protein in the sample. The mixtures were incubated for 3 h at 37°C, pH 4.5-6.0 to enzymatically remove N-linked gycosylation prior to HPLC analysis. About 50 µg of protein was then subject to hydrophobic interaction chromatography (Agilent 1100 HPLC) using an HIC-phenyl column and a high-to-low salt gradient over 35 min. The gradient was achieved using high salt buffer A: 4 M ammonium sulphate containing 20 mM potassium phosphate, pH 6.75; and low salt buffer B: 20 mM potassium phosphate, pH 6.75. Peaks were detected at UV 222 nm. Fractions were collected and analyzed using mass spectroscopy to identify the protein(s) in each peak. Protein ratios are reported as the percent of each peak area relative to the total integrated area of the sample.

### Cloning and Expression of Fv3C:

Fv3C sequence (SEQ ID NOs: 1 and 2) was obtained from the *Fusarium verticillioides* genome in the Broad Institute database (http://www.broadinstitute.org/). The Fv3C open reading frame was amplified by PCR using purified genomic DNA from *Fusarium verticillioides* as the template. The PCR thermocycler used was DNA Engine Tetrad 2 Peltier Thermal Cycler (Bio-Rad Laboratories). The DNA polymerase used was PfuUltra II Fusion HS DNA Polymerase (Stratagene). The primers used to amplify the open reading frame were as follows:
Forward primer MH234 (5'-CACCATGAAGCTGAATTGGGTCGC-3') (SEQ ID NO:15)
Reverse primer MH235 (5'-TTACTCCAACTTGGCGCTG-3') (SEQ ID NO:16)

The forward primers included four additional nucleotides (sequences - CACC) at the 5'-end to facilitate directional cloning into pENTR/D-TOPO (Invitrogen, Carlsbad, CA). The PCR conditions for amplifying the open reading frames were as follows: Step 1: 94°C for 2 minutes. Step 2: 94°C for 30 seconds. Step 3: 57°C for 30 seconds. Step 4: 72°C for 60 seconds. Steps 2, 3 and 4 were repeated for an additional 29 cycles. Step 5: 72°C for 2 min. The PCR product of the Fv3C open reading frame was purified using a Qiaquick PCR Purification Kit (Qiagen). The purified PCR product was initially cloned into the pENTR/D-TOPO vector, transformed into TOP10 Chemically Competent *E. coli* cells (Invitrogen) and plated on LA plates containing 50 ppm kanamycin. Plasmid DNA was obtained from the *E. coli* transformants using a QIAspin plasmid preparation kit (Qiagen). Sequence confirmation for the DNA inserted in the pENTR/D-TOPO vector was obtained using M13 forward and reverse primers and the following additional sequencing primers:
MH255 (5'-AAGCCAAGAGCTTTGTGTCC-3') (SEQ ID NO:17)
MH256 (5'-TATGCACGAGCTCTACGCCT-3') (SEQ ID NO:18)
MH257 (5'-ATGGTACCCTGGCTATGGCT-3') (SEQ ID NO:19)
MH258 (5'-CGGTCACGGTCTATCTTGGT-3') (SEQ ID N0:20)

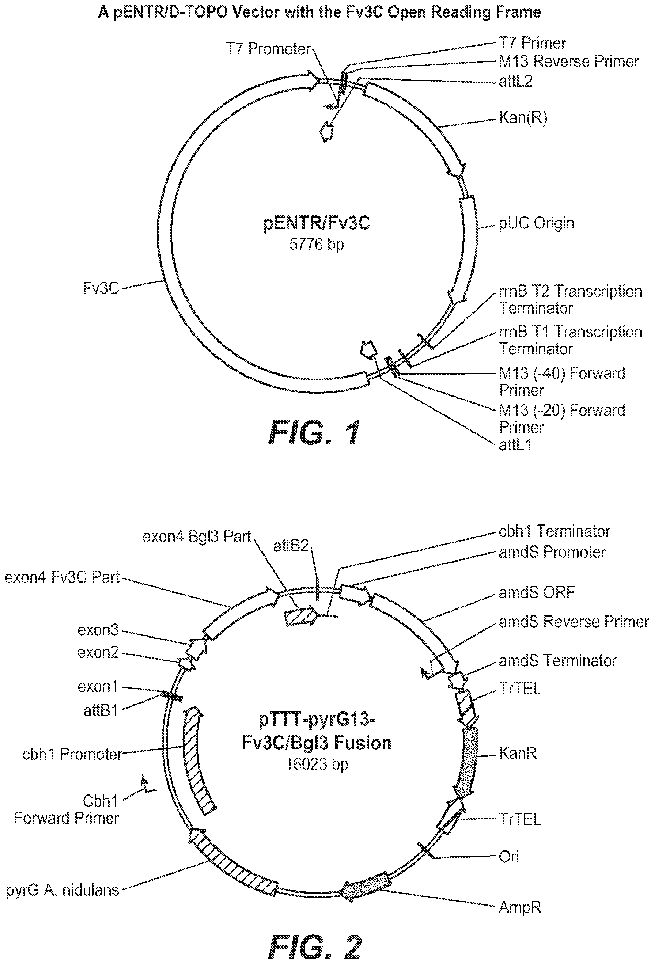

The pENTR/D-TOPO vector with the DNA sequence of the Fv3C open reading frame is depicted in **Figure 1****.**

CHIMERIC β-GLUCOSIDASE: Portions of the wild type *Fusarium verticillioides* Fv3C (SEQ ID NOs: 1 and 2) C-terminal gene sequence were replaced with C-terminal sequence from *T. reesei* β-glucosidase, Bgl3 (or Tr3B) (SEQ ID NOs: 7 and 8). Specifically, a contiguous stretch representing residues 1-691 of Fv3C was fused with a contiguous stretch representing residues 668-874 of Bgl3 (SEQ ID NOs: 9 and 10). The chimeric/fusion molecule was constructed using fusion PCR. pENTR clones of the genomic Fv3C (**Figure 1**) and Bgl3 coding sequences were used as PCR templates. Both entry clones were constructed in the pDONR™221 vector (Invitrogen). The fusion product was assembled in two steps. First, the Fv3C chimeric part was amplified in a PCR reaction using a pENTR_Fv3C DNA as a template and the following oligonucleotide primers:
pDonor Forward: 5'-GCTAGCATGGATGTTTTCCCAGTCACGACGTTGTAAAACGACGGC- 3' (SEQ ID NO:21)
Fv3C/Bgl3 reverse:5'-GGAGGTTGGAGAACTTGAACGTCGACCAAGATAGACCGTGA CCGAAC TCGTAG 3' (SEQ ID NO:22)

The Bgl3 chimeric part was amplified from a pENTR_Bgl3 vector using the following oligonucleotide primers:
pDonor Reverse: 5'-TGCCAGGAAACAGCTATGACCATGTAATACGACTCACTATAGG-3' (SEQ ID NO:23)
Fv3C/Bgl3 forward: 5'-CTACGAGTTCGGTCACGGTCTATCTTGGTCGACGTTCAAGTTC TCCAACCTCC-3' (SEQ ID NO:24)

In the second step, equimolar of the PCR products (about 1 µL and 0.2 µL of the initial PCR reactions, respectively) were added as templates for a subsequent fusion PCR reaction using a set of nested primers as follows:
Att L1 forward: 5'
   TAAGCTCGGGCCCCAAATAATGATTTTATTTTGACTGATAGT 3' (SEQ ID NO:25)
AttL2 rev.:
   5'GGGATATCAGCTGGATGGCAAATAATGATTTTATTTTGACTGATA 3' (SEQ ID NO:26)

The PCR reactions were performed using a high fidelity Phusion DNA polymerase (Finnzymes OY). The resulting fused PCR product contained the intact Gateway-specific attL1, attL2 recombination sites on the ends, allowing for direct cloning into a final destination vector via a Gateway LR recombination reaction (Invitrogen).

After separation of the DNA fragments on a 0.8% agarose gel, the fragments were purified using a Nucleospin® Extract PCR clean-up kit (Macherey-Nagel GmbH & Co. KG) and 100 ng of each fragment was recombined using a pTTT-pyrG13 destination vector and the LR clonase™ II enzyme mix (Invitrogen). The resulting recombination products were transformed into *E.coli* Max Efficiency DH5α (Invitrogen), and clones containing the expression construct pTTT-pyrG13-Fv3C/Bgl3 fusion (**Figure 2**) containing the chimeric β-glucosidase were selected on 2xYT agar plates, prepared using 16 g/L Bacto Tryptone (Difco), 10 g/L Bacto Yeast Extract (Difco), 5 g/L NaCl, 16 g/L Bacto Agar (Difco), and 100 µg/mL ampicillin. The bacteria were grown in 2x YT medium containing 100 µg/mL of ampicillin. Thereafter, the plasmids were isolated and subject to restriction digests by either BglI or EcoRV. The resulting Fv3C/Bgl3 region was sequenced using an ABI3100 sequence analyzer (Applied Biosystems) for confirmation.

A further chimeric β-glucosidase was constructed, which comprised the N-terminal sequence derived from Fv3C (SEQ ID NOs: 1 and 2), a loop region derived from the sequence of a β-glucosidase from *Talaromyces emersonii* Te3A (SEQ ID NOs: 5 and 6), and a C-terminal part sequence derived from *T. reesei* Bgl3 (Tr3B) (SEQ ID NOs: 7 and 8). This was accomplished by replacing a loop region of the Fv3C/Bgl3 chimera. Specifically Fv3C residues 665 - 683 of the Fv3C/Bgl3 chimera (having a sequence of RRSPSTDGKSSPNN TAAPL (SEQ ID NO:27) were replaced with Te3A residues 634 - 640 (KYNITPI (SEQ ID NO:28))). This hybrid molecule was constructed using a fusion PCR approach.

Two N-glycosylation sites, namely S725N and S751N, were introduced into the Fv3C/Bgl3 backbone. These glycosylation mutations were introduced in the Fv3C/Bgl3 backbone using the fusion PCR amplification technique as described above, employing the pTTT-pyrG13-Fv3C/Bgl3 fusion plasmid **(****Figure 2****)** as a template to generate the initial PCR fragments. The following pairs of primers were used in separate PCR reactions:
Pr CbhI forward: 5' CGGAATGAGCTAGTAGGCAAAGTCAGC 3' (SEQ ID NO:29) and
725/751 reverse: 5'-CTCCTTGATGCGGCGAACGTTCTTGGGGAAGCCATAGTCCTTAA GGTTCTTGCTGAAGTTGCCCAGAGAG 3' (SEQ ID NO:30)
725/751 forward: 5'-GGCTTCCCCAAGAACGTTCGCCGCATCAAGGAGTTTATCTACC CCTACCTGAACACCACTACCTC 3' (SEQ ID NO:31), and
Ter CbhI reverse: 5' GATACACGAAGAGCGGCGATTCTACGG 3' (SEQ ID NO:32).

Next, the PCR fragments were fused using the Pr CbhI forward and Ter CbhI primers. The resulting fusion product included the two desired glycosylation sites, but also contained intact attB1 and attB2 sites, which allowed for recombination with the pDONR221 vector using the Gateway BP recombination reaction (Invitrogen). This resulted in a pENTR-Fv3C/Bgl3/S725N S751N clone, which was then used as a backbone for constructing the triple hybrid molecule Fv3C/Te3A/Bgl3.

To replace the loop of the Fv3C/Bgl3 hybrid at residues 665 - 683 with the loop sequence from Te3A (SEQ ID NOs: 5 and 6), primary PCR reactions were performed using the following primer sets:
Set 1: pDonor Forward: 5'-GCTAGCATGGATGTTTTCCCAGTCACGACGTTGTAAA ACGACGGC 3' (SEQ ID NO:21) and
Te3A reverse: 5'-GATAGACCGTGACCGAACTCGTAGATAGGCGTGATGTT GTACTTGTCGAAGTGACGGTAGTCGATGAAGAC 3' (SEQ ID NO:33);
Set 2 : Te3A2 forward: 5'-GTCTTCATCGACTACCGTCACTTCGACAAGTACAACATCAC GCCTATCTACGAGTTCGGTCACGGTCTATC-3' (SEQ ID NO:34); and
pDonor Reverse: 5'
   TGCCAGGAAACAGCTATGACCATGTAATACGACTCACTATAGG 3' (SEQ ID NO:23)

Fragments obtained in the primary PCR reactions were then fused using the following primers:
Att L1 forward: 5'
   TAAGCTCGGGCCCCAAATAATGATTTTATTTTGACTGATAGT 3' (SEQ ID NO:25) and
AttL2 reverse:
   5'GGGATATCAGCTGGATGGCAAATAATGATTTTATTTTGACTGATA 3' (SEQ ID NO:26).

The resulting PCR product contained the intact Gateway-specific attL1, attL2 recombination sites on the ends, allowing for direct cloning into a final destination vector using a Gateway LR recombination reaction (Invitrogen).

The DNA sequence of the Fv3C/Te3A/Bgl3 encoding gene is listed in SEQ ID NO:11. The amino acid sequence of the Fv3C/Te3A/Bgl3 hybrid is listed in SEQ ID NO:12. The gene sequence encoding the Fv3C/Te3A/Bgl3 chimera was cloned in the pTTT-pyrG13 vector and expressed in a *T. reesei* recipient strain as described below.

Specifically, 0.5-1 µg of this fragment was transformed into the *T. reesei* hexa-delete strain mad6 (*cel7B, cel5A, cel6A, cel7A, cel3A, cel12A* genes deleted, WO 2010/141779) using the PEG-protoplast method with slight modifications as described below. For protoplast preparation, spores were grown for 16-24 h at 24°C in *Trichoderma* Minimal Medium MM, which contained 20 g/L glucose, 15 g/L KH₂PO₄, pH 4.5, 5 g/L (NH₄)₂SO₄, 0.6 g/L MgSO₄x7H₂O, 0.6 g/L CaCl₂x2H₂O, 1 mL of 1000 X *T. reesei* Trace elements solution (which contained 5 g/L FeSO₄x7H₂O, 1.4 g/L ZnSO₄x7H₂O, 1.6 g/L MnSO₄x H₂O, 3.7 g/L COCl₂ x 6H₂O) with shaking at 150 rpm. Germinating spores were harvested by centrifugation and treated with 50 mg/mL of Glucanex G200 (Novozymes AG) solution to lyse the fungal cell walls. Further preparation of the protoplasts was performed in accordance with a method described by Penttilä et al. Gene 61(1987)155-164. The transformation mixtures, which contained about 1 µg of DNA and 1-5x 10⁷ protoplasts in a total volume of 200 µL, were each treated with 2 mL of 25% PEG solution, diluted with 2 volumes of 1.2 M sorbitol/10 mM Tris, pH7.5, 10 mM CaCl₂, mixed with 3% selective top agarose MM containing 5 mM uridine and 20 mM acetamide. The resulting mixtures were poured onto 2% selective agarose plate containing uridine and acetamide. Plates were incubated further for 7-10 d at 28°C before single transformants were re-picked onto fresh MM plates containing uridine and acetamide. Spores from independent clones were used to inoculate a fermentation medium in either 96-well microtiter plates or shake flasks.

### Construction of the β-glucosidase expression vector:

The N-terminal portion of the native *T. reesei* β-glucosidase gene *bgl1* was codon optimized (DNA 2.0, Menlo Park, CA). This synthesized portion comprised the first 447 bases of the coding region of this enzyme. This fragment was then amplified by PCR using primers SK943 and SK941. The remaining region of the native *bgl1* gene was PCR amplified from a genomic DNA sample extracted from *T. reesei* strain RL-P37 (Sheir-Neiss, G et al. Appl. Microbiol. Biotechnol. 1984, 20:46-53), using the primers SK940 and SK942. These two PCR fragments of the *bgl1* gene were fused together in a fusion PCR reaction, using primers SK943 and SK942:
Forward Primer SK943: (5'- CACCATGAGATATAGAACAGCTGCCGCT-3') (SEQ ID NO:35)
Reverse Primer SK941: (5'-CGACCGCCCTGCGGAGTCTTGCCCAGTGGTCCCGCGACAG-3') (SEQ ID NO:36)
Forward Primer (SK940): (5'-CTGTCGCGGGACCACTGGGCAAGACTCCGCAGGGCGGTCG-3') (SEQ ID NO:37)
Reverse Primer (SK942): (5'- CCTACGCTACCGACAGAGTG-3') (SEQ ID NO:38)

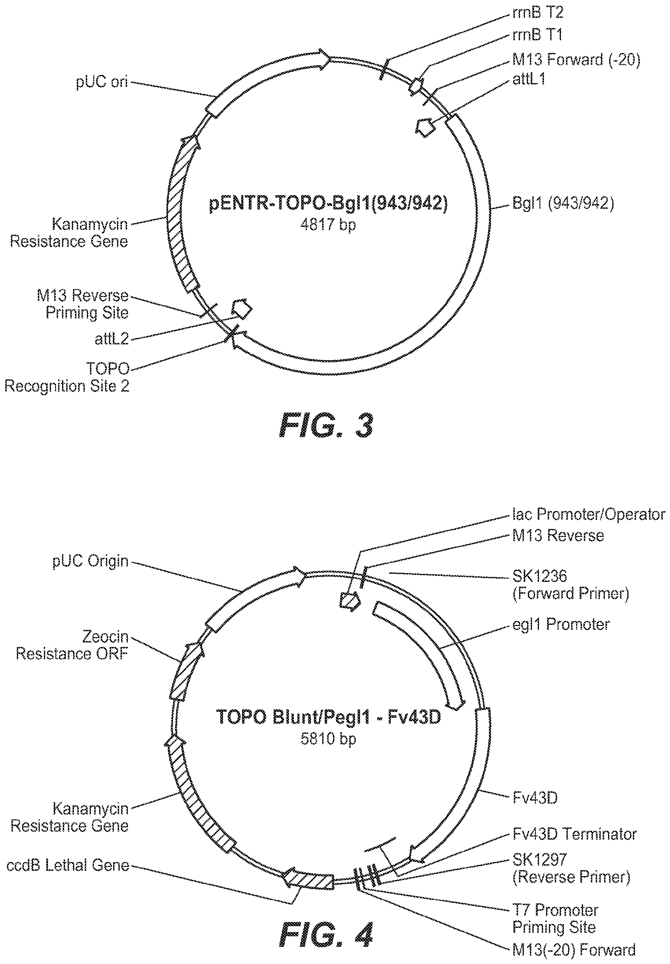

The resulting fusion PCR fragments were cloned into the Gateway ® Entry vector pENTR™/D-TOPO®, and transformed into *E. coli One Shot*® *TOP10* Chemically Competent cells (Invitrogen) resulting in the intermediate vector, pENTR TOPO-Bgl1(943/942) (**Figure 3**). The nucleotide sequence of the inserted DNA was determined. The pENTR-943/942 vector with the correct *bgl1* sequence was recombined with pTrex3g using a LR clonase® reaction (*see,* protocols outlined by Invitrogen). The LR clonase reaction mixture was transformed into *E. coli One Shot*® *TOP10* Chemically Competent cells (Invitrogen), resulting in the expression vector, pTrex3g 943/942. The vector also contained the *Aspergillus nidulans amdS* gene, encoding acetamidase, as a selectable marker for transformation of *T. reesei.* The expression cassette was amplified by PCR with primers SK745 and SK771 (below) to generate the product for transformation of the hexa-delete *T. reesei* strain mad6.
Forward Primer SK771: (5' - GTCTAGACTGGAAACGCAAC -3') (SEQ ID NO:39)
Reverse Primer SK745: (5' - GAGTTGTGAAGTCGGTAATCC -3') (SEQ ID NO:40)

### Construction of the β-xylosidase Fv43D expression cassette:

For the construction of the β-xylosidase Fv43D (SEQ ID NO:13) expression cassette, the *fv43D* gene product (SEQ ID NO:14) was amplified from a *F.verticillioides* genomic DNA sample using the primers SK1322 and SK1297 (below). A region of the promoter of the endoglucanase gene *egl1* was PCR amplified from a *T. reesei* genomic DNA sample extracted from strain RL-P37, using the primers SK1236 and SK1321 (below). These PCR amplified DNA fragments were subsequently fused in a fusion PCR reaction using the primers SK1236 and SK1297 (below). The resulting fusion PCR fragment was cloned into pCR-Blunt II-TOPO vector (Invitrogen) to produce the plasmid TOPO Blunt/Pegl1-Fv43D (*see,* **Figure 4**). This plasmid was then used to transform *E. coli One Shot*® *TOP10* Chemically Competent cells (Invitrogen). The plasmid DNA was extracted from several *E. coli* clones and their sequences were confirmed by restriction digests. The expression cassette was amplified by PCR from the TOPO Blunt/Pegl1-Fv43D using primers SK1236 and SK1297 to generate the product for transformation.
Forward Primer SK1322: (5'-CACCATGCAGCTCAAGTTTCTGTC-3') (SEQ ID NO:41)
Reverse Primer SK1297: (5'-GGTTACTAGTCAACTGCCCGTTCTGTAGCGAG-3') (SEQ ID NO:42)
Forward Primer SK1236: (5'-CATGCGATCGCGACGTTTTGGTCAGGTCG-3') (SEQ ID NO:43)
Reverse Primer SK1321: (5'-GACAGAAACTTGAGCTGCATGGTGTGGGACAACAAGAAGG-3') (SEQ ID NO:44)

### Mixed strain fermentation to produce a beta-xylosidase and beta-glucosidase protein product:

Two hexa-delete *T. reesei* strains expressing either *fv43D* or *fv3C*/*te3A*/*bgl3* were each inoculated into a 250 mL glass 4-baffle flask containing 30 mL of YEG broth (5 g/L yeast extract, 20 g/L glucose). Following 2 days of growth at 28°C, with shaking, the cultures were transferred, in duplicate, to protein production media. The production media was 36 mL of defined broth containing glucose/sophorose and 2 g/L uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100g/L of CaCl₂, 2.5 ml/L *of T. reesei* trace elements (400X)): 175g/L Citric acid anhydrous; 200g/L FeSO₄•7H₂O; 16g/L ZnSO₄•7H₂O; 3.2 g/L CuSO₄•5H₂O; 1.4 g/L MnSO₄•H₂O; 0.8 g/L H₃BO₃ in 250 ml Thomson Ultra Flasks. Transfer volumes were as follows:
Controls, (Fv3C/Te3A/Bgl3) = 4 mL; Fv43D = 4 mL
Test Flasks, Fv43D/ (Fv3C/Te3A/Bgl3) = 2 mL/ 4 mL

All flasks were incubated at 30°C, 160 rpm for four days. After four days incubation, the cells were spun out by centrifuging and the supernatants stored at 4°C pending analysis.

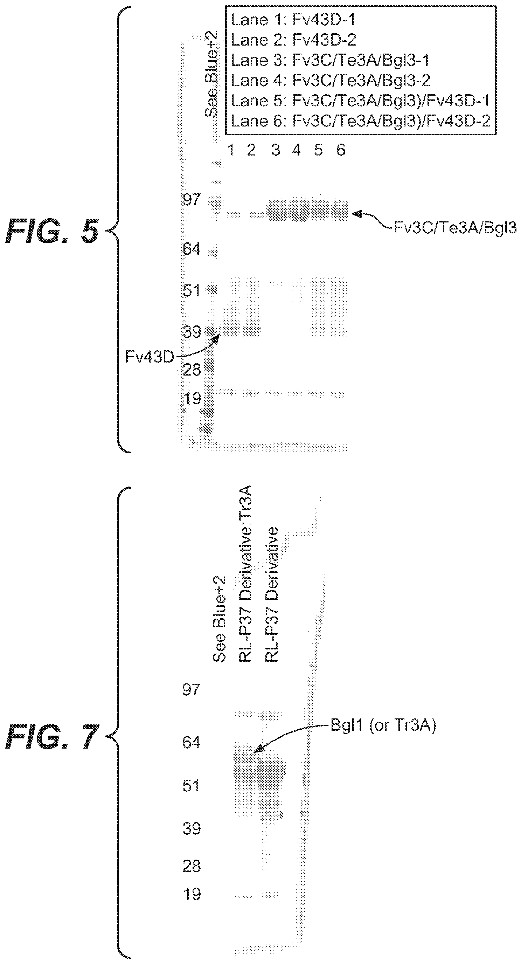

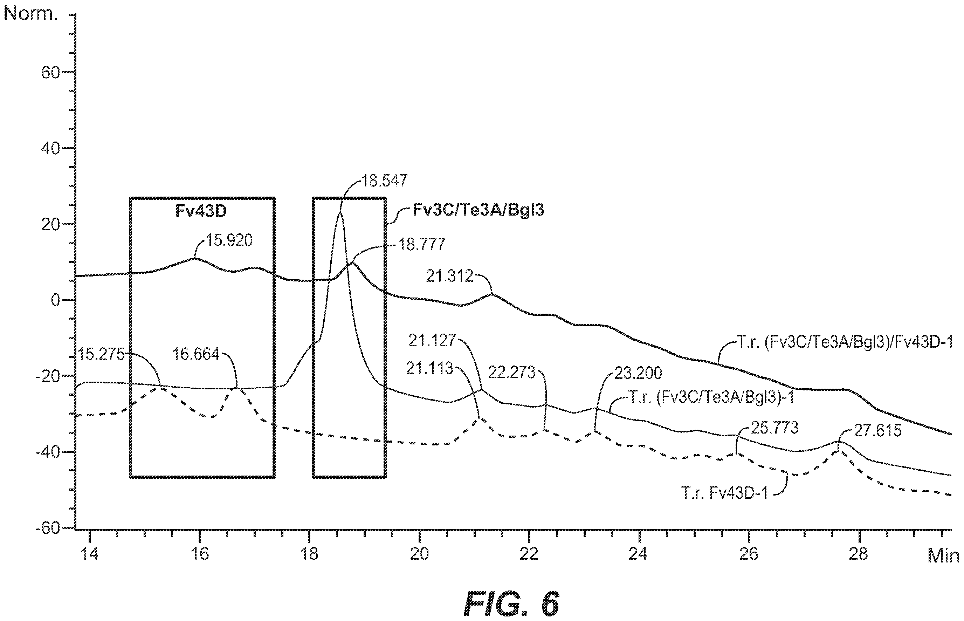

Protein expression in shake flask supernatants was analyzed by SDS-PAGE **(****Figure 5****)** and HPLC **(****Figure 6****)**.

**Table 1. The amount of each protein in the single culture or in the co-culture broth (expressed as the percent of the total integrated area by HPLC).**

| | Percent of total integrated area | |
|---|---|---|
| | Fv43D | Fv3C/Te3A/Bgl3 |
| Fv43D strain single culture product | 46 | 0 |
| (Fv3C/Te3A/Bgl3) strain single culture product | 0 | 64 |
| Fv43D and (Fv3C/Te3A/Bgl3) strain co-culture product | 25 | 16 |

### Mixed strain fermentation to produce a T. reesei protein product with enhanced beta-glucosidase content:

A *Trichoderma reesei* mutant strain (RL-P37-d), derived from RL-P37 (Sheir-Neiss, G. et al.Appl. Microbiol. Biotechnol. 1984, 20:46-53) and selected for high cellulase production was inoculated into one 250 mL glass 4-baffle flask containing 30 mL of YEG broth (5 g/L yeast extract, 20 g/L glucose). A hexa-delete *T. reesei* strain expressing *T. reesei bgl1 (tr3A)* (strain construction was described above, under **Construction of the β-glucosidase expression vector**) was inoculated into a separate 250 mL glass 4-baffle flask containing 30 mL of YEG broth. Following 2 days of growth at 28°C, with shaking, the cultures were transferred to protein production media.

The production media was 36 mL of defined broth containing glucose/sophorose and uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100g/L of CaCl₂, 2.5 ml/L of *T. reesei* trace elements (400X)): 175g/L Citric acid anhydrous; 200g/L FeSO₄•7H₂O; 16g/L ZnSO₄•7H₂O; 3.2 g/L CuSO₄•5H₂O; 1.4 g/L MnSO₄•H₂O; 0.8 g/L H₃BO₃, in 250 mL Thomson Ultra Flasks. Transfer volumes were as follows:
Control, RL-P37-d = 4 mL
Test Flask, RL-P37-d/Tr3A = 4 mL/2 mL

All flasks were incubated at 30°C, 160 rpm for four days. After four days incubation, the cells were spun out by centrifuging and the supernatants stored at 4°C pending analysis.

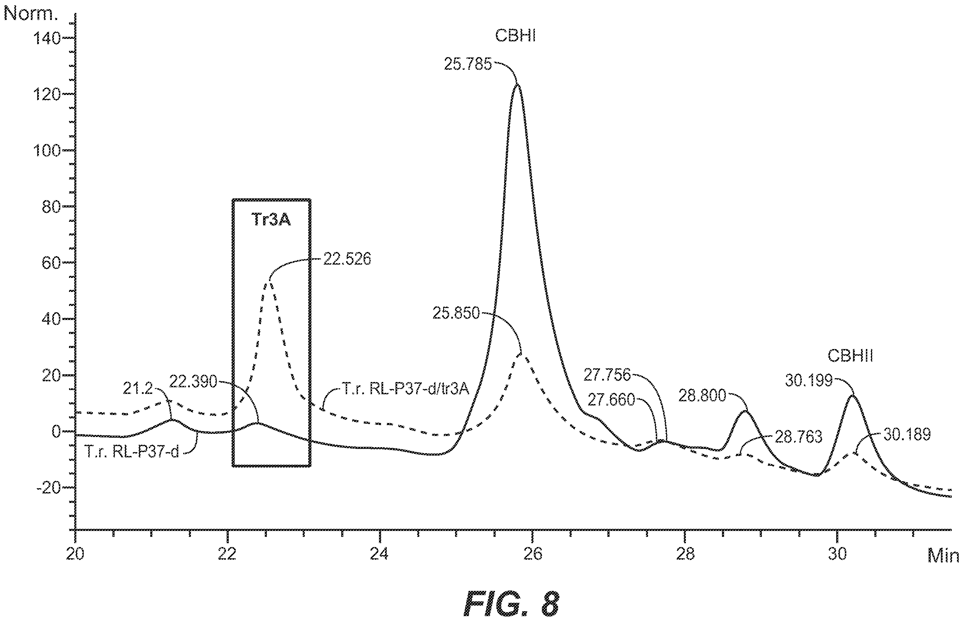

Protein expression in shake flask supernatants was analyzed by SDS-PAGE (Figure 7) and HPLC (**Figure 8**).

**Table 2. The amount of each protein in the single culture or in the co-culture broth (expressed as the percent of the total integrated area by HPLC).**

| | Percent of total integrated area | |
|---|---|---|
| | RL-P37 derivative | Tr3A (Bgl1) |
| RL-P37-d strain single culture product | 98 | 2 |
| RL-P37-d and Tr3A(Bgl1) strain single culture product | 60 | 40 |

### Example 2: Co-fermentation of two Bacillus strains for producing a mixture of amylase variants.

***Materials and Methods:*** Both strains used in this example are production strains. Amylase variant one **(Amylase-1),** a *Bacilllus licheniformis* amylase with four substitutions, is expressed in accordance with US 5,958,739. Amylase variant two **(Amylase-2),** a *Geobacillus stearothermophilus* with a substitution is expressed in accordance with US 20090314286A1. The experiment was conducted in 250 mL Thomson Ultra shake flasks. The seed culture was grown in a seed medium containing yeast extract, phosphate salts, sulfate salts, and other nutritional components, and a suitable defoamer. The control and test flasks used a medium containing lactose, yeast extract, sulfonate, and phosphate salts, other salts, and Maltrin® M-1000 and a suitable antifoam agent. A recipe of such a medium is below:
**Creating an Amylase Blend by Mixed Strain Fermentation:** Two seed cultures were started. A frozen vial of Amylase-1 and Amylase-2 were used to start seed cultures in 35 mls of seed medium in 250 ml baffled glass flasks. The seed flasks were incubated at 38°C, 160 rpm for three hours. After three hours, the seed cultures were used to start a control flask of each enzyme and duplicate test flasks. Each of these flasks had 27 mls of medium. The control flasks were inoculated with 3.0 mls from their respective seed culture. The test flasks were inoculated with 1.5 mls from each seed culture. All flasks were then incubated at 40°C and 160 rpm for 48 hours. At the end of 48 hours incubation, the pH was checked to determine whether the fermentations had reached an end point. All flasks were between pH 7-8 indicating that the endpoint had been reached. An SDS-PAGE gel of supernatants of control and test cultures showed that enzyme had been expressed in all flasks. The duplicate test cultures were analyzed by HPLC to confirm that they contained a blend of both Amylase-1 and Amylase-2. The HPLC did find that the enzymes had co-expressed.

### Example 3: Co-fermentation of two Bacillus strains for producing a mixture of amylase variants with an activity ratio of 1:3

**14L Amylase-1/ Amylase-2 Experiment:** Using the same two enzymes as the flask experiment above, a14L fermentation was run to test whether a certain ratio of the two enzymes could be prepared in a well-controlled manner by co-fermentation. It was decided to create a product with an activity ratio of 1:3 Amylase-1: Amylase-2.

Based on the production rates of the two enzymes at 14L scale, a seed flask (250 mL baffled glass with 30 mL of a seed medium (containing yeast extract, phosphate salts, sulfate salts, and other nutritional components, and a suitable defoamer) was inoculated with 0.2 mL Amylase-1 and 0.8 mL Amylase-2. The seed flask was incubated for 3 hours at 37°C, at 160 rpm. At the end of three hours the entire contents of the flask was transferred to a 14L seed fermenter running with production medium.

When the Oxygen Uptake Rate (OUR) of the seed tank reached 60 mM/Kg/Hr, 0.6 Kgs were used to inoculate the production fermenter, which was run under typical fed batch conditions for producing amylases during a 100 hour fermentation.

Time course samples taken during the production fermentation were assayed for amylase activity. The growth and enzyme production curves were those of a typical 14L scale amylase fermentation. The final 100-hour time point sample was analyzed by HPLC to determine the ratio of the two enzymes. Based on peak area, the protein ratio was 1:2.9 Amylase-1: Amylase-2. Enzymes from the protein backbone, which was used to build the Amylase-2 production strain, show double peaks by this HPLC method. The areas of both peaks are combined to determine the total Amylase-2 area.

### Example 4: Mixed strain fermentation to produce a glucoamylase and two beta-glucosidase protein product

A first hexa-delete *T. reesei* strain expressing *fv3C*/*te3A*/*bgl3*, a second hexa-delete *T. reesei* strain expressing *T. reesei* beta-glucosidase 1 *(bgl1)*; and a quad-deleted *T. reesei* strain (WO 2005/001036) expressing glucoamylase were each inoculated into a 250 mL glass 4-baffle flask containing 30 mL of YEG broth (5 g/L yeast extract, 20 g/L glucose) and 2 g/L uridine. Inocula were taken from sporulated cultures growing on PDA (potato dextrose agar) with uridine. Following 2 days of growth at 28°C, with shaking, the cultures were transferred, in duplicate, to protein production media. Each of the production media was 36 mL of defined broth containing glucose/sophorose and 2 g/L uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100 g/L of CaCl₂, 2.5 mL/L *of T. reesei* trace elements (400X)): 175 g/L Citric acid anhydrous; 200 g/L FeSO₄•7H₂O; 16 g/L ZnSO₄•7H₂O; 3.2 g/L CuSO₄•5H₂O; 1.4 g/L MnSO₄•H₂O; 0.8 g/L H₃BO₃, [placed in a 250 mL glass 4-baffle shake flask. Transfer volumes were as follows:
Controls, (Fv3C/Te3A/Bgl3) = 3 mL; Tr3A = 3 mL; glucoamylase = 3 mL
Test Flask, 1 mL each of (Fv3C/Te3A/Bgl3), Tr3A, glucoamylase.

All flasks were incubated at 28°C, 200 rpm (Innova 4900) for four days. After four days incubation, the cells were removed by centrifugation and the supernatants stored at 4°C pending analysis.

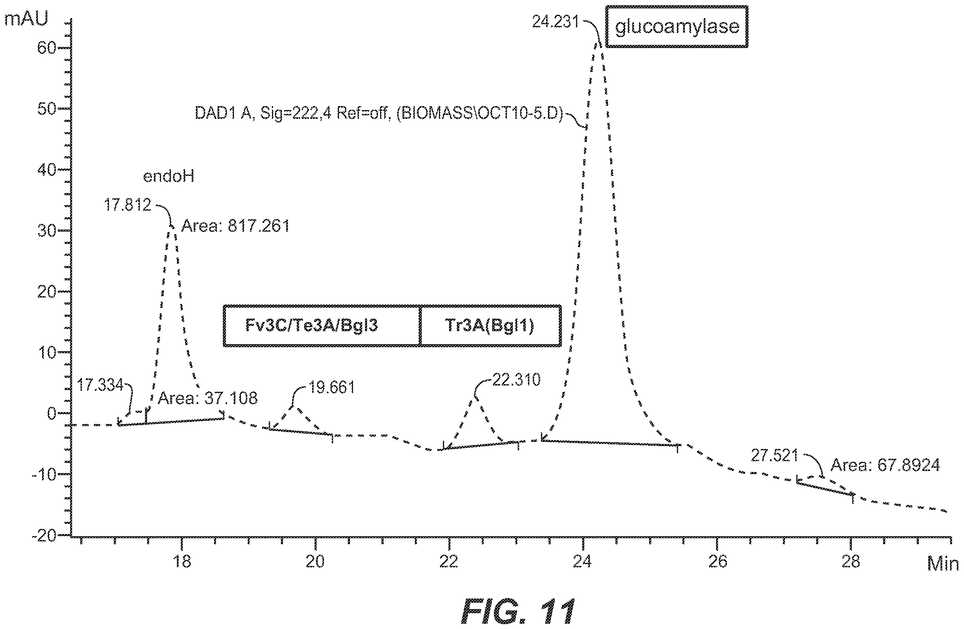

Protein expression in shake flask supernatants was analyzed by SDS-PAGE and HPLC **(****Figure 11****).**

**Table 3. The amount of each protein in the single culture or in the co-culture broth (expressed as the percent of the total integrated area by HPLC).**

| | Percent of total integrated area | | |
|---|---|---|---|
| | (Fv3C/Te3A/Bgl3) | Tr3A | glucoamylase |
| (Fv3C/Te3A/Bgl3) strain single culture product | 69.7 | | |
| Tr3A(Bgl1) strain single culture product | | 65.5 | |
| glucoamylase strain single culture product | | | 96.8 |
| (Fv3C/Te3A/Bgl3), Tr3A(Bgl1), and glucoamylase co-culture product | 3.2 | 6.7 | 86.5 |

### Example 5: Mixed strain fermentation to produce a T. reesei protein product with enhanced β-glucosidase content by direct inoculation

A *T. reesei* mutant strain (RL-P37-d), derived from RL-P37 (Sheir-Neiss, G. et al. Appl. Microbiol. Biotechnol. 1984, 20:46-53) and selected for high cellulase production was incubated with a hexa-delete strain expressing *fv3C*/*te3A*/*bgl3.* Each strain was inoculated into a single 250 ml glass 4-baffle flask containing 30 mls production media, with glucose/sophorose and uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100g/L of CaCl₂, 2.5 ml/L of *T. reesei* trace elements (400X)): 175g/L Citric acid anhydrous; 200g/L FeSO₄•7H₂O; 16g/L ZnSO₄•7H₂O; 3.2 g/L CuSO₄•5H₂O; 1.4 g/L MnSO₄•H₂O; 0.8 g/L H₃BO₃, from mature, sporulated PDA plates with uridine. Following 4 days of growth at 28°C, with shaking, the cultures were harvested. Cells were removed by centrifugation and the supernatants stored at 4°C pending analysis by HPLC. In the mixed culture broth, (Fv3C/Te3A/Bgl3) represented 56% of the total protein, whereas it represented 70% of the total protein when the Fv3C/Te3A/Bgl3 strain was grown separately as a single culture.

### Example 6: Mixed strain fermentation to produce a T. reesei protein product with two β-glucosidases, glucoamylase, and beta-xylosidase content by direct inoculation

A first hexa-delete *T. reesei* strain expressing *fv3C*/*te3A*/*bgl3*, a second hexa-delete *T. reesei* strain expressing *bgl1 (tr3A)*, a third hexa-delete strain expressing *fv43D*, and a quad-deleted *T. reesei* strain (WO 2005/001036) expressing *T.reesei* glucoamylase were each inoculated into a single 250 mL glass 4-baffle flask containing 30 mL of production media with glucose/sophorose and uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100g/L of CaCl₂, 2.5 ml/L *of T. reesei* trace elements (400X)): 175g/L Citric acid anhydrous; 200g/L FeSO₄•7H₂O; 16g/L ZnSO₄•7H₂O; 3.2 g/L CuSO₄•5H₂O; 1.4 g/L MnSO₄•H₂O; 0.8 g/L H₃BO₃, from mature, sporulated PDA plates with uridine. Following 4 days of growth at 28°C, with shaking, the cultures were harvested. Cells were removed by centrifugation and the supernatants stored at 4°C pending analysis by HPLC.

**Table 4. The amount of each protein in the single culture or in the co-culture broth (expressed as the percent of the total integrated area by HPLC). ***

| | Percent of total integrated area | | | |
|---|---|---|---|---|
| | Fv43D | (Fv3C/Te3A/ Bgl3) | Tr3A | glucoamylase |
| Fv43D strain single culture product | 30.3 | | | |
| (Fv3C/Te3A/Bgl3) strain single culture product | | 69.7 | | |
| Tr3A (Bgl1) strain single culture product | | | 65.5 | |
| glucoamylase strain single culture product | | | | 96.8 |
| Fv43D, (Fv3C/Te3A/Bgl3), Tr3A(Bgl1), and glucoamylase co-culture product | 2.3 | 2.2 | 15.8 | 73.5 |

| | | | | |
|---|---|---|---|---|
| * The single cultures were all cultured with an additional step of a starter culture, whereas the mixed culture (last row) was inoculated directly from agar plugs, without a starter culture step. | | | | |

Although preferred methods and materials have been described, any methods and materials similar or equivalent to those described herein can be used in the practice or testing of the present invention. Unless otherwise apparent from the context, any embodiment, aspect, step, feature, element or limitation can be used in combination with any other.

### SEQUENCE LISTING

SEQ ID NO:1 **Nucleotide sequence of Fv3C, a GH3 family β-glucosidase from *Fusarium verticillioides*** 
ATGAAGCTGAATTGGGTCGCCGCAGCCCTGTCTATAGGTGCTGCTGGCACTGACAG
CGCAGTTGCTCTTGCTTCTGCAGTTCCAGACACTTTGGCTGGTGTAAAGGTCAGTTT
TTTTCACCATTTCCTCGTCTAATCTCAGCCTTGTTGCCATATCGCCCTTGTTCGCTC
GGACGCCACGCACCAGATCGCGATCATTTCCTCCCTTGCAGCCTTGGTTCCTCTTAC
GATCTTCCCTCCGCAATTATCAGCGCCCTTAGTCTACACAAAAACCCCCGAGACAGT
CTTTCATTGAGTTTGTCGACATCAAGTTGCTTCTCAACTGTGCATTTGCGTGGCTGTC
TACTTCTGCCTCTAGACAACCAAATCTGGGCGCAATTGACCGCTCAAACCTTGTTCA
AATAACCTTTTTATTCGAGACGCACATTTATAAATATGCGCCTTTCAATAATACCG
ACTTTATGCGCGGCGGCTGCTGTGGCGGTTGATCAGAAAGCTGACGCTCAAAAGGT
TGTCACGAGAGATACACTCGCATACTCGCCGCCTCATTATCCTTCACCATGGATGGA
CCCTAATGCTGTTGGCTGGGAGGAAGCTTACGCCAAAGCCAAGAGCTTTGTGTCCC
AACTCACTCTCATGGAAAAGGTCAACTTGACCACTGGTGTTGGGTAAGCAGCTCCTT
GCAAACAGGGTATCTCAATCCCCTCAGCTAACAACTTCTCAGATGGCAAGGCGAAC
GCTGTGTAGGAAACGTGGGATCAATTCCTCGTCTCGGTATGCGAGGTCTCTGTCTCC
AGGATGGTCCTCTTGGAATTCGTCTGTCCGACTACAACAGCGCTTTTCCCGCTGGCA
CCACAGCTGGTGCTTCTTGGAGCAAGTCTCTCTGGTATGAGAGAGGTCTCCTGATGG
GCACTGAGTTCAAGGAGAAGGGTATCGATATCGCTCTTGGTCCTGCTACTGGACCTC
TTGGTCGCACTGCTGCTGGTGGACGAAACTGGGAAGGCTTCACCGTTGATCCTTATA
TGGCTGGCCACGCCATGGCCGAGGCCGTCAAGGGTATTCAAGACGCAGGTGTCATT
GCTTGTGCTAAGCATTACATCGCAAACGAGCAGGGTAAGCCACTTGGACGATTTGA
GGAATTGACAGAGAACTGACCCTCTTGTAGAGCACTTCCGACAGAGTGGCGAGGTC
CAGTCCCGCAAGTACAACATCTCCGAGTCTCTCTCCTCCAACCTGGATGACAAGACT
ATGCACGAGCTCTACGCCTGGCCCTTCGCTGACGCCGTCCGCGCCGGCGTCGGTTCC
GTCATGTGCTCGTACAACCAGATCAACAACTCGTACGGTTGCCAGAACTCCAAGCT
CCTCAACGGTATCCTCAAGGACGAGATGGGCTTCCAGGGTTTCGTCATGAGCGATT
GGGCGGCCCAGCATACCGGTGCCGCTTCTGCCGTCGCTGGTCTCGATATGAGCATGC
CTGGTGACACTGCCTTCGACAGCGGATACAGCTTCTGGGGCGGAAACTTGACTCTG
GCTGTCATCAACGGAACTGTTCCCGCCTGGCGAGTTGATGACATGGCTCTGCGAATC
ATGTCTGCCTTCTTCAAGGTTGGAAAGACGATAGAGGATCTTCCCGACATCAACTTC
TCCTCCTGGACCCGCGACACCTTCGGCTTCGTGCATACATTTGCTCAAGAGAACCGC
GAGCAGGTCAACTTTGGAGTCAACGTCCAGCACGACCACAAGAGCCACATCCGTGA
GGCCGCTGCCAAGGGAAGCGTCGTGCTCAAGAACACCGGGTCCCTTCCCCTCAAGA
ACCCAAAGTTCCTCGCTGTCATTGGTGAGGACGCCGGTCCCAACCCTGCTGGACCCA
ATGGTTGTGGTGACCGTGGTTGCGATAATGGTACCCTGGCTATGGCTTGGGGCTCGG
GAACTTCCCAATTCCCTTACTTGATCACCCCCGATCAAGGGCTCTCTAATCGAGCTA
CTCAAGACGGAACTCGATATGAGAGCATCTTGACCAACAACGAATGGGCTTCAGTA
CAAGCTCTTGTCAGCCAGCCTAACGTGACCGCTATCGTTTTCGCCAATGCCGACTCT
GGTGAGGGATACATTGAAGTCGACGGAAACTTTGGTGATCGCAAGAACCTCACCCT
CTGGCAGCAGGGAGACGAGCTCATCAAGAACGTGTCGTCCATATGCCCCAACACCA
TTGTAGTTCTGCACACCGTCGGCCCTGTCCTACTCGCCGACTACGAGAAGAACCCCA
ACATCACTGCCATCGTCTGGGCTGGTCTTCCCGGCCAAGAGTCAGGCAATGCCATCG
CTGATCTCCTCTACGGCAAGGTCAGCCCTGGCCGATCTCCCTTCACTTGGGGCCGCA
CCCGCGAGAGCTACGGTACTGAGGTTCTTTATGAGGCGAACAACGGCCGTGGCGCT
CCTCAGGATGACTTCTCTGAGGGTGTCTTCATCGACTACCGTCACTTCGACCGACGA
TCTCCAAGCACCGATGGAAAGAGCTCTCCCAACAACACCGCTGCTCCTCTCTACGA
GTTCGGTCACGGTCTATCTTGGTCCACCTTTGAGTACTCTGACCTCAACATCCAGAA
GAACGTCGAGAACCCCTACTCTCCTCCCGCTGGCCAGACCATCCCCGCCCCAACCTT
 
TGGCAACTTCAGCAAGAACCTCAACGACTACGTGTTCCCCAAGGGCGTCCGATACA
TCTACAAGTTCATCTACCCCTTCCTCAACACCTCCTCATCCGCCAGCGAGGCATCCA
ACGATGGTGGCCAGTTTGGTAAGACTGCCGAAGAGTTCCTCCCTCCCAACGCCCTCA
ACGGCTCAGCCCAGCCTCGTCTTCCCGCCTCTGGTGCCCCAGGTGGTAACCCTCAAT
TGTGGGACATCTTGTACACCGTCACAGCCACAATCACCAACACAGGCAACGCCACC
TCCGACGAGATTCCCCAGCTGTATGTCAGCCTCGGTGGCGAGAACGAGCCCATCCG
TGTTCTCCGCGGTTTCGACCGTATCGAGAACATTGCTCCCGGCCAGAGCGCCATCTT
CAACGCTCAATTGACCCGTCGCGATCTGAGTAACTGGGATACAAATGCCCAGAACT
GGGTCATCACTGACCATCCCAAGACTGTCTGGGTTGGAAGCAGCTCTCGCAAGCTG
CCTCTCAGCGCCAAGTTGGAGTAAGAAAGCCAAACAAGGGTTGTTTTTTGGACTGC
AATTTTTTGGGAGGACATAGTAGCCGCGCCAGTTACGTC
SEQ ID NO:2 **Protein sequence of Fv3C, a GH3 family β-glucosidase from *Fusarium verticillioides*** 
MKLNWVAAALSIGAAGTDSAVALASAVPDTLAGVKKADAQKVVTRDTLAYSPPHYPS
PWMDPNAVGWEEAYAKAKSFVSQLTLMEKVNLTTGVGWQGERCVGNVGSIPRLGMR
GLCLQDGPLGIRLSDYNSAFPAGTTAGASWSKSLWYERGLLMGTEFKEKGIDIALGPAT
GPLGRTAAGGRNWEGFTVDPYMAGHAMAEAVKGIQDAGVIACAKHYIANEQEHFRQS
GEVQSRKYNISESLSSNLDDKTMHELYAWPFADAVRAGVGSVMCSYNQINNSYGCQN
SKLLNGILKDEMGFQGFVMSDWAAQHTGAASAVAGLDMSMPGDTAFDSGYSFWGGN
LTLAVINGTVPAWRVDDMALRIMSAFFKVGKTIEDLPDINFSSWTRDTFGFVHTFAQEN
REQVNFGVNVQHDHKSHIREAAAKGSVVLKNTGSLPLKNPKFLAVIGEDAGPNPAGPN
GCGDRGCDNGTLAMAWGSGTSQFPYLITPDQGLSNRATQDGTRYESILTNNEWASVQA
LVSQPNVTAIVFANADSGEGYIEVDGNFGDRKNLTLWQQGDELIKNVSSICPNTIVVLH
TVGPVLLADYEKNPNITAIVWAGLPGQESGNAIADLLYGKVSPGRSPFTWGRTRESYGT
EVLYEANNGRGAPQDDFSEGVFIDYRHFDRRSPSTDGKSSPNNTAAPLYEFGHGLSWST
FEYSDLNIQKNVENPYSPPAGQTIPAPTFGNFSKNLNDYVFPKGVRYIYKFIYPFLNTSSS
ASEASNDGGQFGKTAEEFLPPNALNGSAQPRLPASGAPGGNPQLWDILYTVTATITNTG
NATSDEIPQLYVSLGGENEPIRVLRGFDRIENIAPGQSAIFNAQLTRRDLSNWDTNAQNW
VITDHPKTVWVGSSSRKLPLSAKLE
SEQ ID NO:3 **Nucleotide sequence of Bgl1 (or Tr3A), a GH3 family β-glucosidase from *Trichoderma reesei*** 
ATGCGTTACCGAACAGCAGCTGCGCTGGCACTTGCCACTGGGCCCTTTGCTAGGGC
AGACAGTCAGTATAGCTGGTCCCATACTGGGATGTGATATGTATCCTGGAGACACC
ATGCTGACTCTTGAATCAAGGTAGCTCAACATCGGGGGCCTCGGCTGAGGCAGTTG
TACCTCCTGCAGGGACTCCATGGGGAACCGCGTACGACAAGGCGAAGGCCGCATTG
GCAAAGCTCAATCTCCAAGATAAGGTCGGCATCGTGAGCGGTGTCGGCTGGAACGG
CGGTCCTTGCGTTGGAAACACATCTCCGGCCTCCAAGATCAGCTATCCATCGCTATG
CCTTCAAGACGGACCCCTCGGTGTTCGATACTCGACAGGCAGCACAGCCTTTACGCC
GGGCGTTCAAGCGGCCTCGACGTGGGATGTCAATTTGATCCGCGAACGTGGACAGT
TCATCGGTGAGGAGGTGAAGGCCTCGGGGATTCATGTCATACTTGGTCCTGTGGCTG
GGCCGCTGGGAAAGACTCCGCAGGGCGGTCGCAACTGGGAGGGCTTCGGTGTCGAT
CCATATCTCACGGGCATTGCCATGGGTCAAACCATCAACGGCATCCAGTCGGTAGG
CGTGCAGGCGACAGCGAAGCACTATATCCTCAACGAGCAGGAGCTCAATCGAGAA
ACCATTTCGAGCAACCCAGATGACCGAACTCTCCATGAGCTGTATACTTGGCCATTT
GCCGACGCGGTTCAGGCCAATGTCGCTTCTGTCATGTGCTCGTACAACAAGGTCAAT
ACCACCTGGGCCTGCGAGGATCAGTACACGCTGCAGACTGTGCTGAAAGACCAGCT
 
GGGGTTCCCAGGCTATGTCATGACGGACTGGAACGCACAGCACACGACTGTCCAAA
GCGCGAATTCTGGGCTTGACATGTCAATGCCTGGCACAGACTTCAACGGTAACAAT
CGGCTCTGGGGTCCAGCTCTCACCAATGCGGTAAATAGCAATCAGGTCCCCACGAG
CAGAGTCGACGATATGGTGACTCGTATCCTCGCCGCATGGTACTTGACAGGCCAGG
ACCAGGCAGGCTATCCGTCGTTCAACATCAGCAGAAATGTTCAAGGAAACCACAAG
ACCAATGTCAGGGCAATTGCCAGGGACGGCATCGTTCTGCTCAAGAATGACGCCAA
CATCCTGCCGCTCAAGAAGCCCGCTAGCATTGCCGTCGTTGGATCTGCCGCAATCAT
TGGTAACCACGCCAGAAACTCGCCCTCGTGCAACGACAAAGGCTGCGACGACGGGG
CCTTGGGCATGGGTTGGGGTTCCGGCGCCGTCAACTATCCGTACTTCGTCGCGCCCT
ACGATGCCATCAATACCAGAGCGTCTTCGCAGGGCACCCAGGTTACCTTGAGCAAC
ACCGACAACACGTCCTCAGGCGCATCTGCAGCAAGAGGAAAGGACGTCGCCATCGT
CTTCATCACCGCCGACTCGGGTGAAGGCTACATCACCGTGGAGGGCAACGCGGGCG
ATCGCAACAACCTGGATCCGTGGCACAACGGCAATGCCCTGGTCCAGGCGGTGGCC
GGTGCCAACAGCAACGTCATTGTTGTTGTCCACTCCGTTGGCGCCATCATTCTGGAG
CAGATTCTTGCTCTTCCGCAGGTCAAGGCCGTTGTCTGGGCGGGTCTTCCTTCTCAG
GAGAGCGGCAATGCGCTCGTCGACGTGCTGTGGGGAGATGTCAGCCCTTCTGGCAA
GCTGGTGTACACCATTGCGAAGAGCCCCAATGACTATAACACTCGCATCGTTTCCGG
CGGCAGTGACAGCTTCAGCGAGGGACTGTTCATCGACTATAAGCACTTCGACGACG
CCAATATCACGCCGCGGTACGAGTTCGGCTATGGACTGTGTAAGTTTGCTAACCTGA
ACAATCTATTAGACAGGTTGACTGACGGATGACTGTGGAATGATAGCTTACACCAA
GTTCAACTACTCACGCCTCTCCGTCTTGTCGACCGCCAAGTCTGGTCCTGCGACTGG
GGCCGTTGTGCCGGGAGGCCCGAGTGATCTGTTCCAGAATGTCGCGACAGTCACCG
TTGACATCGCAAACTCTGGCCAAGTGACTGGTGCCGAGGTAGCCCAGCTGTACATC
ACCTACCCATCTTCAGCACCCAGGACCCCTCCGAAGCAGCTGCGAGGCTTTGCCAA
GCTGAACCTCACGCCTGGTCAGAGCGGAACAGCAACGTTCAACATCCGACGACGAG
ATCTCAGCTACTGGGACACGGCTTCGCAGAAATGGGTGGTGCCGTCGGGGTCGTTT
GGCATCAGCGTGGGAGCGAGCAGCCGGGATATCAGGCTGACGAGCACTCTGTCGGT
AGCGTAG
SEQ ID NO:4 **Protein sequence *of T. reesei* beta glucosidase 1 (Bgl1, a GH3 family β-glucosidase from *Trichoderma reesei*** 
MRYRTAAALALATGPFARADSHSTSGASAEAVVPPAGTPWGTAYDKAKAALAKLNLQ
DKVGIVSGVGWNGGPCVGNTSPASKISYPSLCLQDGPLGVRYSTGSTAFTPGVQAAST
WDVNLIRERGQFIGEEVKASGIHVILGPVAGPLGKTPQGGRNWEGFGVDPYLTGIAMG
QTINGIQSVGVQATAKHYILNEQELNRETISSNPDDRTLHELYTWPFADAVQANVASVM
CSYNKVNTTWACEDQYTLQTVLKDQLGFPGYVMTDWNAQHTTVQSANSGLDMSMPG
TDFNGNNRLWGPALTNAVNSNQVPTSRVDDMVTRILAAWYLTGQDQAGYPSFNISRN
VQGNHKTNVRAIARDGIVLLKNDANILPLKKPASIAVVGSAAIIGNHARNSPSCNDKGC
DDGALGMGWGSGAVNYPYFVAPYDAINTRASSQGTQVTLSNTDNTSSGASAARGKDV
AIVFITADSGEGYITVEGNAGDRNNLDPWHNGNALVQAVAGANSNVIVVVHSVGAIILE
QILALPQVKAVVWAGLPSQESGNALVDVLWGDVSPSGKLVYTIAKSPNDYNTRIVSGG
SDSFSEGLFIDYKHFDDANITPRYEFGYGLSYTKFNYSRLSVLSTAKSGPATGAVVPGGP
SDLFQNVATVTVDIANSGQVTGAEVAQLYITYPSSAPRTPPKQLRGFAKLNLTPGQSGT
ATFNIRRRDLSYWDTASQKWVVPSGSFGISVGASSRDIRLTSTLSVA
SEQ ID NO:5 **Nucleotide sequenced of Te3A, a GH3 family β-glucosidase from *Talaromyces emersonii,* codon-optimized for expression in *T.reesei*** 
ATGCGCAACGGCCTCCTCAAGGTCGCCGCCTTAGCCGCTGCCAGCGCCGTCAACGG
CGAGAACCTCGCCTACAGCCCCCCTTCTACCCCAGCCCCTGGGCCAACGGCCAGG
GCGACTGGGCCGAGGCCTACCAGAAGGCCGTCCAGTTCGTCAGCCAGCTCACCCTC
GCCGAGAAGGTCAACCTCACCACCGGCACCGGCTGGGAGCAGGACCGCTGCGTCGG
CCAGGTCGGCAGCATCCCCCGCTTAGGCTTCCCCGGCCTCTGCATGCAGGACAGCCC
CCTCGGCGTCCGCGACACCGACTACAACAGCGCCTTCCCTGCCGGCGTTAACGTCGC
CGCCACCTGGGACCGCAACTTAGCCTACCGCAGAGGCGTCGCCATGGGCGAGGAAC
ACCGCGGCAAGGGCGTCGACGTCCAGTTAGGCCCCGTCGCCGGCCCCTTAGGCCGC
TCTCCTGATGCCGGCCGCAACTGGGAGGGCTTCGCCCCCGACCCCGTCCTCACCGGC
AACATGATGGCCAGCACCATCCAGGGCATCCAGGATGCTGGCGTCATTGCCTGCGC
CAAGCACTTCATCCTCTACGAGCAGGAACACTTCCGCCAGGGCGCCCAGGACGGCT
ACGACATCAGCGACAGCATCAGCGCCAACGCCGACGACAAGACCATGCACGAGTT
ATACCTCTGGCCCTTCGCCGATGCCGTCCGCGCCGGTGTCGGCAGCGTCATGTGCAG
CTACAACCAGGTCAACAACAGCTACGCCTGCAGCAACAGCTACACCATGAACAAGC
TCCTCAAGAGCGAGTTAGGCTTCCAGGGCTTCGTCATGACCGACTGGGGCGGCCAC
CACAGCGGCGTCGGCTCTGCCCTCGCCGGCCTCGACATGAGCATGCCCGGCGACAT
TGCCTTCGACAGCGGCACGTCTTTCTGGGGCACCAACCTCACCGTTGCCGTCCTCAA
CGGCTCCATCCCCGAGTGGCGCGTCGACGACATGGCCGTCCGCATCATGAGCGCCT
ACTACAAGGTCGGCCGCGACCGCTACAGCGTCCCCATCAACTTCGACAGCTGGACC
CTCGACACCTACGGCCCCGAGCACTACGCCGTCGGCCAGGGCCAGACCAAGATCAA
CGAGCACGTCGACGTCCGCGGCAACCACGCCGAGATCATCCACGAGATCGGCGCCG
CCTCCGCCGTCCTCCTCAAGAACAAGGGCGGCCTCCCCCTCACTGGCACCGAGCGCT
TCGTCGGTGTCTTTGGCAAGGATGCTGGCAGCAACCCCTGGGGCGTCAACGGCTGC
AGCGACCGCGGCTGCGACAACGGCACCCTCGCCATGGGCTGGGGCAGCGGCACCGC
CAACTTTCCCTACCTCGTCACCCCCGAGCAGGCCATCCAGCGCGAGGTCCTCAGCCG
CAACGGCACCTTCACCGGCATCACCGACAACGGCGCCTTAGCCGAGATGGCCGCTG
CCGCCTCTCAGGCCGACACCTGCCTCGTCTTTGCCAACGCCGACTCCGGCGAGGGCT
ACATCACCGTCGATGGCAACGAGGGCGACCGCAAGAACCTCACCCTCTGGCAGGGC
GCCGACCAGGTCATCCACAACGTCAGCGCCAACTGCAACAACACCGTCGTCGTCTT
ACACACCGTCGGCCCCGTCCTCATCGACGACTGGTACGACCACCCCAACGTCACCG
CCATCCTCTGGGCCGGTTTACCCGGTCAGGAAAGCGGCAACAGCCTCGTCGACGTC
CTCTACGGCCGCGTCAACCCCGGCAAGACCCCCTTCACCTGGGGCAGAGCCCGCGA
CGACTATGGCGCCCCTCTCATCGTCAAGCCTAACAACGGCAAGGGCGCCCCCCAGC
AGGACTTCACCGAGGGCATCTTCATCGACTACCGCCGCTTCGACAAGTACAACATC
ACCCCCATCTACGAGTTCGGCTTCGGCCTCAGCTACACCACCTTCGAGTTCAGCCAG
TTAAACGTCCAGCCCATCAACGCCCCTCCCTACACCCCCGCCAGCGGCTTTACGAAG
GCCGCCCAGAGCTTCGGCCAGCCCTCCAATGCCAGCGACAACCTCTACCCTAGCGA
CATCGAGCGCGTCCCCCTCTACATCTACCCCTGGCTCAACAGCACCGACCTCAAGGC
CAGCGCCAACGACCCCGACTACGGCCTCCCCACCGAGAAGTACGTCCCCCCCAACG
CCACCAACGGCGACCCCCAGCCCATTGACCCTGCCGGCGGTGCCCCTGGCGGCAAC
CCCAGCCTCTACGAGCCCGTCGCCCGCGTCACCACCATCATCACCAACACCGGCAA
GGTCACCGGCGACGAGGTCCCCCAGCTCTATGTCAGCTTAGGCGGCCCTGACGACG
CCCCCAAGGTCCTCCGCGGCTTCGACCGCATCACCCTCGCCCCTGGCCAGCAGTACC
TCTGGACCACCACCCTCACTCGCCGCGACATCAGCAACTGGGACCCCGTCACCCAG
AACTGGGTCGTCACCAACTACACCAAGACCATCTACGTCGGCAACAGCAGCCGCAA
CCTCCCCCTCCAGGCCCCCCTCAAGCCCTACCCCGGCATCTGATGA
SEQ ID NO:6 **Protein sequence of Te3A, a GH3 family β-glucosidase from *Talaromyces emersonii*** 
MRNGLLKVAALAAASAVNGENLAYSPPFYPSPWANGQGDWAEAYQKAVQFVSQLTL
AEKVNLTTGTGWEQDRCVGQVGSIPRLGFPGLCMQDSPLGVRDTDYNSAFPAGVNVA
ATWDRNLAYRRGVAMGEEHRGKGVDVQLGPVAGPLGRSPDAGRNWEGFAPDPVLTG
NMMASTIQGIQDAGVIACAKHFILYEQEHFRQGAQDGYDISDSISANADDKTMHELYL
WPFADAVRAGVGSVMCSYNQVNNSYACSNSYTMNKLLKSELGFQGFVMTDWGGHHS
GVGSALAGLDMSMPGDIAFDSGTSFWGTNLTVAVLNGSIPEWRVDDMAVRIMSAYYK
VGRDRYSVPINFDSWTLDTYGPEHYAVGQGQTKINEHVDVRGNHAEIIHEIGAASAVLL
KNKGGLPLTGTERFVGVFGKDAGSNPWGVNGCSDRGCDNGTLAMGWGSGTANFPYL
VTPEQAIQREVLSRNGTFTGITDNGALAEMAAAASQADTCLVFANADSGEGYITVDGN
EGDRKNLTLWQGADQVIHNVSANCNNTVVVLHTVGPVLIDDWYDHPNVTAILWAGLP
GQESGNSLVDVLYGRVNPGKTPFTWGRARDDYGAPLIVKPNNGKGAPQQDFTEGIFID
YRRFDKYNITPIYEFGFGLSYTTFEFSQLNVQPINAPPYTPASGFTKAAQSFGQPSNASDN
LYPSDIERVPLYIYPWLNSTDLKASANDPDYGLPTEKYVPPNATNGDPQPIDPAGGAPG
GNPSLYEPVARVTTIITNTGKVTGDEVPQLYVSLGGPDDAPKVLRGFDRITLAPGQQYL
WTTTLTRRDISNWDPVTQNWVVTNYTKTIYVGNSSRNLPLQAPLKPYPGI
SEQ ID NO:7 **Nucleotide sequence of Bgl3 (or Tr3B), a GH3 family β-glucosidase from *Trichoderma reesei*** 
ATGAAGACGTTGTCAGTGTTTGCTGCCGCCCTTTTGGCGGCCGTAGCTGAGGCCAAT
CCCTACCCGCCTCCTCACTCCAACCAGGCGTACTCGCCTCCTTTCTACCCTTCGCCAT
GGATGGACCCCAGTGCTCCAGGCTGGGAGCAAGCCTATGCCCAAGCTAAGGAGTTC
GTCTCGGGCTTGACTCTCTTGGAGAAGGTCAACCTCACCACCGGTGTTGGCTGGATG
GGTGAGAAGTGCGTTGGAAACGTTGGTACCGTGCCTCGCTTGGGCATGCGAAGTCT
TTGCATGCAGGACGGCCCCCTGGGTCTCCGATTCAACACGTACAACAGCGCTTTCAG
CGTTGGCTTGACGGCCGCCGCCAGCTGGAGCCGACACCTTTGGGTTGACCGCGGTA
CCGCTCTGGGCTCCGAGGCAAAGGGCAAGGGTGTCGATGTTCTTCTCGGACCCGTG
GCTGGCCCTCTCGGTCGCAACCCCAACGGAGGCCGTAACGTCGAGGGTTTCGGCTC
GGATCCCTATCTGGCGGGTTTGGCTCTGGCCGATACCGTGACCGGAATCCAGAACG
CGGGCACCATCGCCTGTGCCAAGCACTTCCTCCTCAACGAGCAGGAGCATTTCCGCC
AGGTCGGCGAAGCTAACGGTTACGGATACCCCATCACCGAGGCTCTGTCTTCCAAC
GTTGATGACAAGACGATTCACGAGGTGTACGGCTGGCCCTTCCAGGATGCTGTCAA
GGCTGGTGTCGGGTCCTTCATGTGCTCGTACAACCAGGTCAACAACTCGTACGCTTG
CCAAAACTCCAAGCTCATCAACGGCTTGCTCAAGGAGGAGTACGGTTTCCAAGGCT
TTGTCATGAGCGACTGGCAGGCCCAGCACACGGGTGTCGCGTCTGCTGTTGCCGGTC
TCGATATGACCATGCCTGGTGACACCGCCTTCAACACCGGCGCATCCTACTTTGGAA
GCAACCTGACGCTTGCTGTTCTCAACGGCACCGTCCCCGAGTGGCGCATTGACGAC
ATGGTGATGCGTATCATGGCTCCCTTCTTCAAGGTGGGCAAGACGGTTGACAGCCTC
ATTGACACCAACTTTGATTCTTGGACCAATGGCGAGTACGGCTACGTTCAGGCCGCC
GTCAATGAGAACTGGGAGAAGGTCAACTACGGCGTCGATGTCCGCGCCAACCATGC
GAACCACATCCGCGAGGTTGGCGCCAAGGGAACTGTCATCTTCAAGAACAACGGCA
TCCTGCCCCTTAAGAAGCCCAAGTTCCTGACCGTCATTGGTGAGGATGCTGGCGGCA
ACCCTGCCGGCCCCAACGGCTGCGGTGACCGCGGCTGTGACGACGGCACTCTTGCC
ATGGAGTGGGGATCTGGTACTACCAACTTCCCCTACCTCGTCACCCCCGACGCGGCC
CTGCAGAGCCAGGCTCTCCAGGACGGCACCCGCTACGAGAGCATCCTGTCCAACTA
 
CGCCATCTCGCAGACCCAGGCGCTCGTCAGCCAGCCCGATGCCATTGCCATTGTCTT
TGCCAACTCGGATAGCGGCGAGGGCTACATCAACGTCGATGGCAACGAGGGCGACC
GCAAGAACCTGACGCTGTGGAAGAACGGCGACGATCTGATCAAGACTGTTGCTGCT
GTCAACCCCAAGACGATTGTCGTCATCCACTCGACCGGCCCCGTGATTCTCAAGGAC
TACGCCAACCACCCCAACATCTCTGCCATTCTGTGGGCCGGTGCTCCTGGCCAGGAG
TCTGGCAACTCGCTGGTCGACATTCTGTACGGCAAGCAGAGCCCGGGCCGCACTCC
CTTCACCTGGGGCCCGTCGCTGGAGAGCTACGGAGTTAGTGTTATGACCACGCCCA
ACAACGGCAACGGCGCTCCCCAGGATAACTTCAACGAGGGCGCCTTCATCGACTAC
CGCTACTTTGACAAGGTGGCTCCCGGCAAGCCTCGCAGCTCGGACAAGGCTCCCAC
GTACGAGTTTGGCTTCGGACTGTCGTGGTCGACGTTCAAGTTCTCCAACCTCCACAT
CCAGAAGAACAATGTCGGCCCCATGAGCCCGCCCAACGGCAAGACGATTGCGGCTC
CCTCTCTGGGCAGCTTCAGCAAGAACCTTAAGGACTATGGCTTCCCCAAGAACGTTC
GCCGCATCAAGGAGTTTATCTACCCCTACCTGAGCACCACTACCTCTGGCAAGGAG
GCGTCGGGTGACGCTCACTACGGCCAGACTGCGAAGGAGTTCCTCCCCGCCGGTGC
CCTGGACGGCAGCCCTCAGCCTCGCTCTGCGGCCTCTGGCGAACCCGGCGGCAACC
GCCAGCTGTACGACATTCTCTACACCGTGACGGCCACCATTACCAACACGGGCTCG
GTCATGGACGACGCCGTTCCCCAGCTGTACCTGAGCCACGGCGGTCCCAACGAGCC
GCCCAAGGTGCTGCGTGGCTTCGACCGCATCGAGCGCATTGCTCCCGGCCAGAGCG
TCACGTTCAAGGCAGACCTGACGCGCCGTGACCTGTCCAACTGGGACACGAAGAAG
CAGCAGTGGGTCATTACCGACTACCCCAAGACTGTGTACGTGGGCAGCTCCTCGCG
CGACCTGCCGCTGAGCGCCCGCCTGCCATGA
SEQ ID NO:8 **Protein sequence of Bgl3 (or Tr3B), a GH3 family β-glucosidase from *Trichoderma reesei*** 
MKTLSVFAAALLAAVAEANPYPPPHSNQAYSPPFYPSPWMDPSAPGWEQAYAQAKEF
VSGLTLLEKVNLTTGVGWMGEKCVGNVGTVPRLGMRSLCMQDGPLGLRFNTYNSAFS
VGLTAAASWSRHLWVDRGTALGSEAKGKGVDVLLGPVAGPLGRNPNGGRNVEGFGS
DPYLAGLALADTVTGIQNAGTIACAKHFLLNEQEHFRQVGEANGYGYPITEALSSNVDD
KTIHEVYGWPFQDAVKAGVGSFMCSYNQVNNSYACQNSKLINGLLKEEYGFQGFVMS
DWQAQHTGVASAVAGLDMTMPGDTAFNTGASYFGSNLTLAVLNGTVPEWRIDDMVM
RIMAPFFKVGKTVDSLIDTNFDSWTNGEYGYVQAAVNENWEKVNYGVDVRANHANHI
REVGAKGTVIFKNNGILPLKKPKFLTVIGEDAGGNPAGPNGCGDRGCDDGTLAMEWGS
GTTNFPYLVTPDAALQSQALQDGTRYESILSNYAISQTQALVSQPDAIAIVFANSDSGEG
YINVDGNEGDRKNLTLWKNGDDLIKTVAAVNPKTIVVIHSTGPVILKDYANHPNISAIL
WAGAPGQESGNSLVDILYGKQSPGRTPFTWGPSLESYGVSVMTTPNNGNGAPQDNFNE
GAFIDYRYFDKVAPGKPRSSDKAPTYEFGFGLSWSTFKFSNLHIQKNNVGPMSPPNGKTI
AAPSLGSFSKNLKDYGFPKNVRRIKEFIYPYLSTTTSGKEASGDAHYGQTAKEFLPAGAL
DGSPQPRSAASGEPGGNRQLYDILYTVTATITNTGSVMDDAVPQLYLSHGGPNEPPKVL
RGFDRIERIAPGQSVTFKADLTRRDLSNWDTKKQQWVITDYPKTVYVGSSSRDLPLSAR
LP
SEQ ID NO:9 **The nucleotide sequence encoding Fv3C/Bgl3** 
ATGAAGCTGA ATTGGGTCGC CGCAGCCCTG TCTATAGGTG CTGCTGGCAC
TGACAGCGCA GTTGCTCTTG CTTCTGCAGT TCCAGACACT TTGGCTGGTG
TAAAGGTCAG TTTTTTTTCA CCATTTCCTC GTCTAATCTC AGCCTTGTTG
CCATATCGCC CTTGTTCGCT CGGACGCCAC GCACCAGATC GCGATCATTT
CCTCCCTTGC AGCCTTGGTT CCTCTTACGA TCTTCCCTCC GCAATTATCA
GCGCCCTTAG TCTACACAAA AACCCCCGAG ACAGTCTTTC ATTGAGTTTG
 
TCGACATCAA GTTGCTTCTC AACTGTGCAT TTGCGTGGCT GTCTACTTCT
GCCTCTAGAC AACCAAATCT GGGCGCAATT GACCGCTCAA ACCTTGTTCA
AATAACCTTT TTTATTCGAG ACGCACATTT ATAAATATGC GCCTTTCAAT
AATACCGACT TTATGCGCGG CGGCTGCTGT GGCGGTTGAT CAGAAAGCTG
ACGCTCAAAA GGTTGTCACG AGAGATACAC TCGCATACTC GCCGCCTCAT
TATCCTTCAC CATGGATGGA CCCTAATGCT GTTGGCTGGG AGGAAGCTTA
CGCCAAAGCC AAGAGCTTTG TGTCCCAACT CACTCTCATG GAAAAGGTCA
ACTTGACCAC TGGTGTTGGG TAAGCAGCTC CTTGCAAACA GGGTATCTCA
ATCCCCTCAG CTAACAACTT CTCAGATGGC AAGGCGAACG CTGTGTAGGA
AACGTGGGAT CAATTCCTCG TCTCGGTATG CGAGGTCTCT GTCTCCAGGA
TGGTCCTCTT GGAATTCGTC TGTCCGACTA CAACAGCGCT TTTCCCGCTG
GCACCACAGC TGGTGCTTCT TGGAGCAAGT CTCTCTGGTA TGAGAGAGGT
CTCCTGATGG GCACTGAGTT CAAGGAGAAG GGTATCGATA TCGCTCTTGG
TCCTGCTACT GGACCTCTTG GTCGCACTGC TGCTGGTGGA CGAAACTGGG
AAGGCTTCAC CGTTGATCCT TATATGGCTG GCCACGCCAT GGCCGAGGCC
GTCAAGGGTA TTCAAGACGC AGGTGTCATT GCTTGTGCTA AGCATTACAT
CGCAAACGAG CAGGGTAAGC CACTTGGACG ATTTGAGGAA TTGACAGAGA
ACTGACCCTC TTGTAGAGCA CTTCCGACAG AGTGGCGAGG TCCAGTCCCG
CAAGTACAAC ATCTCCGAGT CTCTCTCCTC CAACCTGGAT GACAAGACTA
TGCACGAGCT CTACGCCTGG CCCTTCGCTG ACGCCGTCCG CGCCGGCGTC
GGTTCCGTCA TGTGCTCGTA CAACCAGATC AACAACTCGT ACGGTTGCCA
GAACTCCAAG CTCCTCAACG GTATCCTCAA GGACGAGATG GGCTTCCAGG
GTTTCGTCAT GAGCGATTGG GCGGCCCAGC ATACCGGTGC CGCTTCTGCC
GTCGCTGGTC TCGATATGAG CATGCCTGGT GACACTGCCT TCGACAGCGG
ATACAGCTTC TGGGGCGGAA ACTTGACTCT GGCTGTCATC AACGGAACTG
TTCCCGCCTG GCGAGTTGAT GACATGGCTC TGCGAATCAT GTCTGCCTTC
TTCAAGGTTG GAAAGACGAT AGAGGATCTT CCCGACATCA ACTTCTCCTC
CTGGACCCGC GACACCTTCG GCTTCGTGCA TACATTTGCT CAAGAGAACC
GCGAGCAGGT CAACTTTGGA GTCAACGTCC AGCACGACCA CAAGAGCCAC
ATCCGTGAGG CCGCTGCCAA GGGAAGCGTC GTGCTCAAGA ACACCGGGTC
CCTTCCCCTC AAGAACCCAA AGTTCCTCGC TGTCATTGGT GAGGACGCCG
GTCCCAACCC TGCTGGACCC AATGGTTGTG GTGACCGTGG TTGCGATAAT
GGTACCCTGG CTATGGCTTG GGGCTCGGGA ACTTCCCAAT TCCCTTACTT
GATCACCCCC GATCAAGGGC TCTCTAATCG AGCTACTCAA GACGGAACTC
GATATGAGAG CATCTTGACC AACAACGAAT GGGCTTCAGT ACAAGCTCTT
GTCAGCCAGC CTAACGTGAC CGCTATCGTT TTCGCCAATG CCGACTCTGG
TGAGGGATAC ATTGAAGTCG ACGGAAACTT TGGTGATCGC AAGAACCTCA
CCCTCTGGCA GCAGGGAGAC GAGCTCATCA AGAACGTGTC GTCCATATGC
CCCAACACCA TTGTAGTTCT GCACACCGTC GGCCCTGTCC TACTCGCCGA
CTACGAGAAG AACCCCAACA TCACTGCCAT CGTCTGGGCT GGTCTTCCCG
GCCAAGAGTC AGGCAATGCC ATCGCTGATC TCCTCTACGG CAAGGTCAGC
CCTGGCCGAT CTCCCTTCAC TTGGGGCCGC ACCCGCGAGA GCTACGGTAC
TGAGGTTCTT TATGAGGCGA ACAACGGCCG TGGCGCTCCT CAGGATGACT
TCTCTGAGGG TGTCTTCATC GACTACCGTC ACTTCGACCG ACGATCTCCA
AGCACCGATG GAAAGAGCTC TCCCAACAAC ACCGCTGCTC CTCTCTACGA
GTTCGGTCAC GGTCTATCTT GGTCGACGTT CAAGTTCTCC AACCTCCACA
TCCAGAAGAA CAATGTCGGC CCCATGAGCC CGCCCAACGG CAAGACGATT
GCGGCTCCCT CTCTGGGCAG CTTCAGCAAG AACCTTAAGG ACTATGGCTT
CCCCAAGAAC GTTCGCCGCA TCAAGGAGTT TATCTACCCC TACCTGAGCA
CCACTACCTC TGGCAAGGAG GCGTCGGGTG ACGCTCACTA CGGCCAGACT
GCGAAGGAGT TCCTCCCCGC CGGTGCCCTG GACGGCAGCC CTCAGCCTCG
 
CTCTGCGGCC TCTGGCGAAC CCGGCGGCAA CCGCCAGCTG TACGACATTC
TCTACACCGT GACGGCCACC ATTACCAACA CGGGCTCGGT CATGGACGAC
GCCGTTCCCC AGCTGTACCT GAGCCACGGC GGTCCCAACG AGCCGCCCAA
GGTGCTGCGT GGCTTCGACC GCATCGAGCG CATTGCTCCC GGCCAGAGCG
TCACGTTCAA GGCAGACCTG ACGCGCCGTG ACCTGTCCAA CTGGGACACG
AAGAAGCAGC AGTGGGTCAT TACCGACTAC CCCAAGACTG TGTACGTGGG
CAGCTCCTCG CGCGACCTGC CGCTGAGCGC CCGCCTGCCA TGA
SEQ ID NO:10 **The Fv3C/Bgl3 chimeric polypeptide sequence (the Bgl3 chimeric part is in bold and upper case)** 
MKLNWVAAALSIGAAGTDSAVALASAVPDTLAGVKKADAQKVVTRDTLAYSPPHYPS
PWMDPNAVGWEEAYAKAKSFVSQLTLMEKVNLTTGVGWQGERCVGNVGSIPRLGMR
GLCLQDGPLGIRLSDYNSAFPAGTTAGASWSKSLWYERGLLMGTEFKEKGIDIALGPAT
GPLGRTAAGGRNWEGFTVDPYMAGHAMAEAVKGIQDAGVIACAKHYIANEQEHFRQS
GEVQSRKYNISESLSSNLDDKTMHELYAWPFADAVRAGVGSVMCSYNQINNSYGCQN
SKLLNGILKDEMGFQGFVMSDWAAQHTGAASAVAGLDMSMPGDTAFDSGYSFWGGN
LTLAVINGTVPAWRVDDMALRIMSAFFKVGKTIEDLPDINFSSWTRDTFGFVHTFAQEN
REQVNFGVNVQHDHKSHIREAAAKGSVVLKNTGSLPLKNPKFLAVIGEDAGPNPAGPN
GCGDRGCDNGTLAMAWGSGTSQFPYLITPDQGLSNRATQDGTRYESILTNNEWASVQA
LVSQPNVTAIVFANADSGEGYIEVDGNFGDRKNLTLWQQGDELIKNVSSICPNTIVVLH
TVGPVLLADYEKNPNITAIVWAGLPGQESGNAIADLLYGKVSPGRSPFTWGRTRESYGT
EVLYEANNGRGAPQDDFSEGVFIDYRHFDRRSPSTDGKSSPNNTAAPLYEFGHGLSWST
FKFSNLHIQKNNVGPMSPPNGKTIAAPSLGSFSKNLKDYGFPKNVRRIKEFIYPYLSTTTS
GKEASGDAHYGQTAKEFLPAGALDGSPQPRSAASGEPGGNRQLYDILYTVTATITNTGS
VMDDAVPQLYLSHGGPNEPPKVLRGFDRIERIAPGQSVTFKADLTRRDLSNWDTKKQQ
WVITDYPKTVYVGSSSRDLPLSARLP
SEQ ID NO:11: Nucleic acid sequence encoding the Fv3C/Te3A/Bgl3 chimera 
ATGAAGCTGAATTGGGTCGCCGCAGCCCTGTCTATAGGTGCTGCTGGCACTGACAG
CGCAGTTGCTCTTGCTTCTGCAGTTCCAGACACTTTGGCTGGTGTAAAGGTCAGTTT
TTTTCACCATTTCCTCGTCTAATCTCAGCCTTGTTGCCATATCGCCCTTGTTCGCTC
GGACGCCACGCACCAGATCGCGATCATTTCCTCCCTTGCAGCCTTGGTTCCTCTTAC
GATCTTCCCTCCGCAATTATCAGCGCCCTTAGTCTACACAAAAACCCCCGAGACAGT
CTTTCATTGAGTTTGTCGACATCAAGTTGCTTCTCAACTGTGCATTTGCGTGGCTGTC
TACTTCTGCCTCTAGACAACCAAATCTGGGCGCAATTGACCGCTCAAACCTTGTTCA
AATAACCTTTTTATTCGAGACGCACATTTATAAATATGCGCCTTTCAATAATACCG
ACTTTATGCGCGGCGGCTGCTGTGGCGGTTGATCAGAAAGCTGACGCTCAAAAGGT
TGTCACGAGAGATACACTCGCATACTCGCCGCCTCATTATCCTTCACCATGGATGGA
CCCTAATGCTGTTGGCTGGGAGGAAGCTTACGCCAAAGCCAAGAGCTTTGTGTCCC
AACTCACTCTCATGGAAAAGGTCAACTTGACCACTGGTGTTGGGTAAGCAGCTCCTT
GCAAACAGGGTATCTCAATCCCCTCAGCTAACAACTTCTCAGATGGCAAGGCGAAC
GCTGTGTAGGAAACGTGGATCAATTCCTCGTCTCGGTATGCGAGGTCTCTGTCTCC
AGGATGGTCCTCTTGGAATTCGTCTGTCCGACTACAACAGCGCTTTTCCCGCTGGCA
CCACAGCTGGTGCTTCTTGGAGCAAGTCTCTCTGGTATGAGAGAGGTCTCCTGATGG
GCACTGAGTTCAAGGAGAAGGGTATCGATATCGCTCTTGGTCCTGCTACTGGACCTC
TTGGTCGCACTGCTGCTGGTGGACGAAACTGGGAAGGCTTCACCGTTGATCCTTATA
TGGCTGGCCACGCCATGGCCGAGGCCGTCAAGGGTATTCAAGACGCAGGTGTCATT
GCTTGTGCTAAGCATTACATCGCAAACGAGCAGGGTAAGCCACTTGGACGATTTGA
 
GGAATTGACAGAGAACTGACCCTCTTGTAGAGCACTTCCGACAGAGTGGCGAGGTC
CAGTCCCGCAAGTACAACATCTCCGAGTCTCTCCTCCAACCTGGATGACAAGACT
ATGCACGAGCTCTACGCCTGGCCCTTCGCTGACGCCGTCCGCGCCGGCGTCGGTTCC
GTCATGTGCTCGTACAACCAGATCAACAACTCGTACGGTTGCCAGAACTCCAAGCT
CCTCAACGGTATCCTCAAGGACGAGATGGGCTTCCAGGGTTTCGTCATGAGCGATT
GGGCGGCCCAGCATACCGGTGCCGCTTCTGCCGTCGCTGGTCTCGATATGAGCATGC
CTGGTGACACTGCCTTCGACAGCGGATACAGCTTCTGGGGCGGAAACTTGACTCTG
GCTGTCATCAACGGAACTGTTCCCGCCTGGCGAGTTGATGACATGGCTCTGCGAATC
ATGTCTGCCTTCTTCAAGGTTGGAAAGACGATAGAGGATCTTCCCGACATCAACTTC
TCCTCCTGGACCCGCGACACCTTCGGCTTCGTGCATACATTTGCTCAAGAGAACCGC
GAGCAGGTCAACTTTGGAGTCAACGTCCAGCACGACCACAAGAGCCACATCCGTGA
GGCCGCTGCCAAGGGAAGCGTCGTGCTCAAGAACACCGGGTCCCTTCCCCTCAAGA
ACCCAAAGTTCCTCGCTGTCATTGGTGAGGACGCCGGTCCCAACCCTGCTGGACCCA
ATGGTTGTGGTGACCGTGGTTGCGATAATGGTACCCTGGCTATGGCTTGGGGCTCGG
GAACTTCCCAATTCCCTTACTTGATCACCCCCGATCAAGGGCTCTCTAATCGAGCTA
CTCAAGACGGAACTCGATATGAGAGCATCTTGACCAACAACGAATGGGCTTCAGTA
CAAGCTCTTGTCAGCCAGCCTAACGTGACCGCTATCGTTTTCGCCAATGCCGACTCT
GGTGAGGGATACATTGAAGTCGACGGAAACTTTGGTGATCGCAAGAACCTCACCCT
CTGGCAGCAGGGAGACGAGCTCATCAAGAACGTGTCGTCCATATGCCCCAACACCA
TTGTAGTTCTGCACACCGTCGGCCCTGTCCTACTCGCCGACTACGAGAAGAACCCCA
ACATCACTGCCATCGTCTGGGCTGGTCTTCCCGGCCAAGAGTCAGGCAATGCCATCG
CTGATCTCCTCTACGGCAAGGTCAGCCCTGGCCGATCTCCCTTCACTTGGGGCCGCA
CCCGCGAGAGCTACGGTACTGAGGTTCTTTATGAGGCGAACAACGGCCGTGGCGCT
CCTCAGGATGACTTCTCTGAGGGTGTCTTCATCGACTACCGTCACTTCGACAAGTAC
AACATCACGCCTATCTACGAGTTCGGTCACGGTCTATCTTGGTCGACGTTCAAGTTC
TCCAACCTCCACATCCAGAAGAACAATGTCGGCCCCATGAGCCCGCCCAACGGCAA
GACGATTGCGGCTCCCTCTCTGGGCAACTTCAGCAAGAACCTTAAGGACTATGGCTT
CCCCAAGAACGTTCGCCGCATCAAGGAGTTTATCTACCCCTACCTGAACACCACTAC
CTCTGGCAAGGAGGCGTCGGGTGACGCTCACTACGGCCAGACTGCGAAGGAGTTCC
TCCCCGCCGGTGCCCTGGACGGCAGCCCTCAGCCTCGCTCTGCGGCCTCTGGCGAAC
CCGGCGGCAACCGCCAGCTGTACGACATTCTCTACACCGTGACGGCCACCATTACC
AACACGGGCTCGGTCATGGACGACGCCGTTCCCCAGCTGTACCTGAGCCACGGCGG
TCCCAACGAGCCGCCCAAGGTGCTGCGTGGCTTCGACCGCATCGAGCGCATTGCTC
CCGGCCAGAGCGTCACGTTCAAGGCAGACCTGACGCGCCGTGACCTGTCCAACTGG
GACACGAAGAAGCAGCAGTGGGTCATTACCGACTACCCCAAGACTGTGTACGTGGG
CAGCTCCTCGCGCGACCTGCCGCTGAGCGCCCGCCTGCCATGA
SEQ ID NO:12: Amino acid sequence of the Fv3C/Te3A/Bgl3 chimera 
MKLNWVAAALSIGAAGTDSAVALASAVPDTLAGVKKADAQKVVTRDTLAYSPPHYPS
PWMDPNAVGWEEAYAKAKSFVSQLTLMEKVNLTTGVGWQGERCVGNVGSIPRLGMR
GLCLQDGPLGIRLSDYNSAFPAGTTAGASWSKSLWYERGLLMGTEFKEKGIDIALGPAT
GPLGRTAAGGRNWEGFTVDPYMAGHAMAEAVKGIQDAGVIACAKHYIANEQEHFRQS
GEVQSRKYNISESLSSNLDDKTMHELYAWPFADAVRAGVGSVMCSYNQINNSYGCQN
SKLLNGILKDEMGFQGFVMSDWAAQHTGAASAVAGLDMSMPGDTAFDSGYSFWGGN
LTLAVINGTVPAWRVDDMALRIMSAFFKVGKTIEDLPDINFSSWTRDTFGFVHTFAQEN
REQVNFGVNVQHDHKSHIREAAAKGSVVLKNTGSLPLKNPKFLAVIGEDAGPNPAGPN
GCGDRGCDNGTLAMAWGSGTSQFPYLITPDQGLSNRATQDGTRYESILTNNEWASVQA
LVSQPNVTAIVFANADSGEGYIEVDGNFGDRKNLTLWQQGDELIKNVSSICPNTIVVLH
TVGPVLLADYEKNPNITAIVWAGLPGQESGNAIADLLYGKVSPGRSPFTWGRTRESYGT
 
EVLYEANNGRGAPQDDFSEGVFIDYRHFDKYNITPIYEFGHGLSWSTFKFSNLHIQKNN
VGPMSPPNGKTIAAPSLGNFSKNLKDYGFPKNVRRIKEFIYPYLNTTTSGKEASGDAHY
GQTAKEFLPAGALDGSPQPRSAASGEPGGNRQLYDILYTVTATITNTGSVMDDAVPQLY
LSHGGPNEPPKVLRGFDRIERIAPGQSVTFKADLTRRDLSNWDTKKQQWVITDYPKTVY
VGSSSRDLPLSARLP
SEQ ID NO:13: **Nucleotide sequence for Fv43D, a GH43D family enzyme from *Fusarium verticilloides*** 
ATGCAGCTCAAGTTTCTGTCTTCAGCATTGTTGCTGTCTTTGACCGGCAATTGCGCTG
CGCAAGACACTAATGATATCCCTCCTCTGATCACCGACCTCTGGTCTGCGGATCCCT
CGGCTCATGTTTTCGAGGGCAAACTCTGGGTTTACCCATCTCACGACATCGAAGCCA
ATGTCGTCAACGGCACCGGAGGCGCTCAGTACGCCATGAGAGATTATCACACCTAT
TCCATGAAGACCATCTATGGAAAAGATCCCGTTATCGACCATGGCGTCGCTCTGTCA
GTCGATGATGTCCCATGGGCCAAGCAGCAAATGTGGGCTCCTGACGCAGCTTACAA
GAACGGCAAATATTATCTCTACTTCCCCGCCAAGGATAAAGATGAGATCTTCAGAA
TTGGAGTTGCTGTCTCCAACAAGCCCAGCGGTCCTTTCAAGGCCGACAAGAGCTGG
ATCCCCGGTACTTACAGTATCGATCCTGCTAGCTATGTCGACACTAATGGCGAGGCA
TACCTCATCTGGGGCGGTATCTGGGGCGGCCAGCTTCAGGCCTGGCAGGATCACAA
GACCTTTAATGAGTCGTGGCTCGGCGACAAAGCTGCTCCCAACGGCACCAACGCCC
TATCTCCTCAGATCGCCAAGCTAAGCAAGGACATGCACAAGATCACCGAGACACCC
CGCGATCTCGTCATCCTGGCCCCCGAGACAGGCAAGCCCCTTCAAGCAGAGGACAA
TAAGCGACGATTTTTCGAGGGGCCCTGGGTTCACAAGCGCGGCAAGCTGTACTACC
TCATGTACTCTACCGGCGACACGCACTTCCTCGTCTACGCGACTTCCAAGAACATCT
ACGGTCCTTATACCTATCAGGGCAAGATTCTCGACCCTGTTGATGGGTGGACTACGC
ATGGAAGTATTGTTGAGTACAAGGGACAGTGGTGGTTGTTCTTTGCGGATGCGCAT
ACTTCTGGAAAGGATTATCTGAGACAGGTTAAGGCGAGGAAGATCTGGTATGACAA
GGATGGCAAGATTTTGCTTACTCGTCCTAAGATTTAG
SEQ ID NO:14 **Protein sequence of Fv43D** 
MQLKFLSSALLLSLTGNCAAQDTNDIPPLITDLWSADPSAHVFEGKLWVYPSHDIEANV
VNGTGGAQYAMRDYHTYSMKTIYGKDPVIDHGVALSVDDVPWAKQQMWAPDAAYK
NGKYYLYFPAKDKDEIFRIGVAVSNKPSGPFKADKSWIPGTYSIDPASYVDTNGEAYLI
WGGIWGGQLQAWQDHKTFNESWLGDKAAPNGTNALSPQIAKLSKDMHKITETPRDLV
ILAPETGKPLQAEDNKRRFFEGPWVHKRGKLYYLMYSTGDTHFLVYATSKNIYGPYTY
QGKILDPVDGWTTHGSIVEYKGQWWLFFADAHTSGKDYLRQVKARKIWYDKDGKILL
TRPKI
SEQ ID NO:15 Forward primer MH234
   5'-CACCATGAAGCTGAATTGGGTCGC-3'
SEQ ID NO:16 Reverse primer MH235
   5'-TTACTCCAACTTGGCGCTG-3'
SEQ ID NO:17 MH255
   5'-AAGCCAAGAGCTTTGTGTCC-3'
SEQ ID NO:18 MH256
   5'-TATGCACGAGCTCTACGCCT-3'
SEQ ID NO:19 MH257
   5'-ATGGTACCCTGGCTATGGCT-3'
SEQ ID NO:20 MH258
   5'-CGGTCACGGTCTATCTTGGT-3'
SEQ ID NO:21 pDonor Forward
   5'- GCTAGCATGGATGTTTTCCCAGTCACGACGTTGTAAAACGACGGC- 3'
SEQ ID NO:22 Fv3C/Bgl3 reverse 
5’-GGAGGTTGGAGAACTTGAACGTCGACCAAGATAGACCGTGA CCGAAC TCGTAG
3’
SEQ ID NO:23 pDonor Reverse
   5'-TGCCAGGAAACAGCTATGACCATGTAATACGACTCACTATAGG-3'
SEQ ID NO:24 Fv3C/Bgl3 forward
   5'- CTACGAGTTCGGTCACGGTCTATCTTGGTCGACGTTCAAGTTC TCCAACCTCC-3'
SEQ ID NO:25 Att L1 forward
   5'TAAGCTCGGGCCCCAAATAATGATTTTATTTTGACTGATAGT 3'
SEQ ID NO:26 AttL2 reverse
   5'GGGATATCAGCTGGATGGCAAATAATGATTTTATTTTGACTGATA 3'
SEQ ID NO:27 Fv3C residues 665 - 683 of the Fv3C/Bgl3 chimera
   RRSPSTDGKSSPNN TAAPL
SEQ ID NO:28 Te3A residues 634 - 640
   KYNITPI
SEQ ID NO:29 Pr CbhI forward
   5' CGGAATGAGCTAGTAGGCAAAGTCAGC 3'
SEQ ID NO:30 725/751 reverse 
5’-CTCCTTGATGCGGCGAACGTTCTTGGGGAAGCCATAGTCCTTAA
GGTTCTTGCTGAAGTTGCCCAGAGAG 3’
SEQ ID NO:31 725/751 forward 
5'- GGCTTCCCCAAGAACGTTCGCCGCATCAAGGAGTTTATCTACC
CCTACCTGAACACCACTACCTC 3'
SEQ ID NO:32 Ter CbhI reverse
   5'GATACACGAAGAGCGGCGATTCTACGG 3'
SEQ ID NO:33 Te3A reverse 
5'-GATAGACCGTGACCGAACTCGTAGATAGGCGTGATGTT
GTACTTGTCGAAGTGACGGTAGTCGATGAAGAC 3'
SEQ ID NO:34 Te3A2 forward 
5'-GTCTTCATCGACTACCGTCACTTCGACAAGTACAACATCAC
GCCTATCTACGAGTTCGGTCACGGTCTATC-3'
SEQ ID NO:35 Forward Primer SK943
   5'- CACCATGAGATATAGAACAGCTGCCGCT-3'
SEQ ID NO:36 Reverse Primer SK941
   5'-CGACCGCCCTGCGGAGTCTTGCCCAGTGGTCCCGCGACAG-3'
SEQ ID NO:37 Forward Primer (SK940)
   5'-CTGTCGCGGGACCACTGGGCAAGACTCCGCAGGGCGGTCG-3'
SEQ ID NO:38 Reverse Primer (SK942)
   5'- CCTACGCTACCGACAGAGTG-3'
SEQ ID NO:39 Forward Primer SK771
   5' - GTCTAGACTGGAAACGCAAC -3'
SEQ ID NO:40 Reverse Primer SK745
   5' - GAGTTGTGAAGTCGGTAATCC -3'
SEQ ID NO:41 Forward Primer SK1322
   5'-CACCA TGCAGCTCAAGTTTCTGTC-3'
SEQ ID NO:42 Reverse Primer SK1297
   5'-GGTTACTAGTCAACTGCCCGTTCTGTAGCGAG-3'
SEQ ID NO:43 Forward Primer SK1236
   5'-CATGCGATCGCGACGTTTTGGTCAGGTCG-3'
SEQ ID NO:44 Reverse Primer SK1321
   5'-GACAGAAACTTGAGCTGCATGGTGTGGGACAACAAGAAGG-3'

## Claims

1. A method of producing a mixture of enzymes catalyzing a conversion process, comprising the steps of:
combining first and second cell lines in a liquid medium, the first cell line encoding and disposed to express a first set of one or more enzymes, the second cell line encoding and disposed to express a second set of one or more enzymes, the first and second sets of enzymes having catalytic activities enhancing the conversion process, one or more of the first set of enzymes being heterologous to the first cell line, and one or more of the second set of enzymes being heterologous to the second cell line;
culturing the combined cell lines; whereby the cell lines secrete the enzymes into the medium, or are lysed releasing the enzymes, thereby providing a mixture of enzymes in proportions effective to enhance the conversion process,
wherein the first and second cell lines are filamentous fungal cell lines or bacterial cell lines.

2. The method of claim 1, wherein one or more of the second set of enzymes is not encoded by the first cell line; or
one or more of the second set of enzymes being expressed at a lower level by the first cell line.

3. The method of claim 1, further comprising:
identifying a plurality of enzymes catalyzing the conversion process;
identifying or constructing a first cell line encoding, and disposed to express a first set of one or more of the identified enzymes, and a second cell line encoding, and disposed to express a second set of the one or more enzymes to provide the first and second cell lines.

4. The method of claim 1, further comprising
identifying or constructing cell lines encoding and disposed to express different sets of enzymes catalyzing a conversion process, the cell lines having been grown under selective pressure or under conditions that allow auxotroph growth to retain their disposure to expressing the enzymes to form a bank of cell lines; wherein the plurality of cell lines comprise the first cell line encoding and disposed to express a first set of one or more enzymes, and the second cell line encoding and disposed to express a second set of one or more enzymes;
selecting the first and second cell lines from the bank.

5. The method of claim 4, wherein the cell lines are grown under different selective pressures in the identifying step and without selective pressure in the culturing step.

6. A method of preparing a cell bank, comprising
identifying a plurality of enzymes catalyzing a conversion process; identifying or constructing a plurality of cell lines encoding and disposed to express different sets of the plurality of enzymes;
propagating the cell lines under different selective pressures or under conditions that allow auxotroph growth to retain disposure to express the set of enzymes encoded by a cell lines, to provide a cell bank; and
combining different combinations of the cells lines, culturing the combined cell lines in a liquid medium, wherein the enzymes are secreted or the cells are lysed and the enzymes released to the medium to provide different mixture of enzymes, and comparing the capacity of the enzymes to enhance the conversion process,
wherein the plurality of enzymes are heterologous to the plurality of cells,
and wherein the plurality of cell lines are filamentous fungal cell lines or bacterial cell lines.

7. The method of any one of claims 1-6, further comprising separating the mixture of enzymes from cells in the culture.

8. The method of any one of claims 1-7, further comprising combining the mixture of enzymes with a substrate, wherein the mixture of enzymes enhances conversion of the substrate to a product.

9. The method of any one of claims 1-8, wherein the substrate is cellulose and/or hemicellulose and the product is glucose, or wherein the substrate is starch and the product is sugar.

10. The method of any one of claims 1-9, wherein the molar ratio of an enzyme of the first set of enzymes and an enzyme of the second set of enzymes in the mixture is at least two-fold different than a molar ratio of the enzymes expressed by either the first or second cell line alone.

11. The method of any one of claims 1-9, wherein the molar ratio of the first set of enzymes that are secreted and the second set of enzymes that are secreted is at least twofold different than a molar ratio of the enzymes expressed by either the first or second cell line alone.

12. The method of any one of claims 1-11, wherein the first and second cell lines are the same strain.

13. The method of any one of claims 1-12, wherein the first and/or second cell lines are Trichoderma reesei cell lines.

14. The method of any one of claims 1-12, wherein the first and/or second cell lines are Bacillus cell lines.

15. The method of any one of claims 1-14, further comprising determining growth profiles of the cell lines before the combining step.

## Patentansprüche

1. Verfahren für die Herstellung einer Mischung von Enzymen, die einen Umwandlungsvorgang katalysieren, umfassend die Schritte des:
Kombinierens erster und zweiter Zelllinien in einem flüssigen Medium, wobei die erste Zelllinie für einen ersten Satz von einem oder mehreren Enzymen kodiert und zum Exprimieren derselben bereit ist, wobei die zweite Zelllinie für einen zweiten Satz von einem oder mehreren Enzymen kodiert und zum Exprimieren derselben bereit ist, wobei die ersten und zweiten Sätze von Enzymen katalytische Aktivitäten aufweisen, die den Umwandlungvorgang verbessern, wobei einer oder mehrere des ersten Satzes von Enzymen heterolog zu der ersten Zelllinie ist und einer oder mehrere des zweiten Satzes von Enzymen heterolog zu der zweiten Zelllinie ist;
Züchtens der kombinierten Zelllinien; wobei die Zelllinien die Enzyme in das Medium absondern oder unter Freisetzen der Enzyme lysiert werden, wodurch eine Mischung von Enzymen in Verhältnissen bereitgestellt wird, die wirksam sind, den Umwandlungsvorgang zu verbessern,
wobei die ersten und zweiten Zelllinien fadenförmige Pilzzelllinien oder Bakterienzelllinien sind.

2. Verfahren nach Anspruch 1, wobei ein oder mehrere von dem zweiten Satz von Enzymen nicht durch die erste Zelllinie kodiert ist/sind; oder
ein oder mehrere von dem zweiten Satzes von Enzymen auf einem niedrigeren Niveau durch die erste Zelllinie exprimiert wird/werden.

3. Verfahren nach Anspruch 1, ferner Folgendes umfassend:
Identifizieren einer Mehrzahl von Enzymen, die den Umwandlungsvorgang katalysieren;
Identifizieren oder Konstruieren einer ersten Zelllinie, die für einen ersten Satz von einem oder mehreren der identifizierten Enzymen kodiert und zum Exprimieren derselben bereit ist, und einer zweiten Zelllinie, die für einen zweiten Satz von einem oder mehreren Enzymen kodiert und zum Exprimieren derselben bereit ist, um die ersten und zweiten Zelllinien bereitzustellen.

4. Verfahren nach Anspruch 1, ferner Folgendes umfassend:
Identifizieren oder Konstruieren von Zelllinien, die für verschiedene Sätze von Enzymen, die einen Umwandlungsvorgang katalysieren, kodieren und zum Exprimieren derselben bereit sind, wobei die Zelllinien unter selektivem Druck oder unter Bedingungen gezüchtet worden sind, die auxotrophes Wachstum gestatten, um ihre Bereitschaft beizubehalten, die Enzyme zum Bilden einer Bank von Zelllinien zu exprimieren; wobei die Mehrzahl von Zelllinien die erste Zelllinie, die für einen ersten Satz von einem oder mehreren Enzymen kodiert und zum Exprimieren derselben bereit ist und die zweite Zelllinie, die für einen zweiten Satz von einem oder mehreren Enzymen kodiert und zum Exprimieren derselben bereit ist, umfasst;
Auswählen der ersten und zweiten Zelllinien aus der Bank.

5. Verfahren nach Anspruch 4, wobei die Zelllinien unter verschiedenen selektiven Drucken im Identifizierungsschritt und ohne selektiven Druck im Züchtungsschritt gezüchtet werden.

6. Verfahren für die Herstellung einer Zellbank, umfassend
Identifizieren einer Mehrzahl von Enzymen, die einen Umwandlungsvorgang katalysieren; Identifizieren oder Konstruieren einer Mehrzahl von Zelllinien, die für verschiedene Sätze der Mehrzahl von Enzymen kodieren und zum Exprimieren derselben bereit sind;
Propagieren der Zelllinien unter verschiedenen selektivem Drucken oder unter Bedingungen, die auxotrophes Wachstum gestatten, um die Bereitschaft beizubehalten, den Satz von Enzymen, die durch eine Zelllinie kodiert werden, zu exprimieren, um eine Zellbank bereitzustellen;
Kombinieren verschiedener Kombinationen der Zelllinien, Züchten der kombinierten Zelllinien in einem flüssigen Medium, wobei die Enzyme abgesondert oder die Zellen lysiert werden und die Enzyme in das Medium freigesetzt werden, um verschiedene Mischungen von Enzymen bereitzustellen und Vergleichen der Fähigkeit der Enzyme, den Umwandlungsvorgang zu verbessern,
wobei die Mehrzahl von Enzymen heterolog zu der Mehrzahl von Zellen sind
und wobei die Mehrzahl von Zelllinien fadenförmige Pilzzelllinien oder Bakterienzelllinien sind.

7. Verfahren nach einem der Ansprüche 1-6, ferner das Trennen der Mischung von Enzymen von Zellen in der Kultur umfassend.

8. Verfahren nach einem der Ansprüche 1-7, ferner das Kombinieren der Mischung von Enzymen mit einem Substrat umfassend, wobei die Mischung von Enzymen die Umwandlung des Substrats zu einem Produkt verbessert.

9. Verfahren nach einem der Ansprüche 1-8, wobei das Substrat Cellulose und/oder Hemicellulose ist und das Produkt Glucose ist oder wobei das Substrat Stärke ist und das Produkt Zucker ist.

10. Verfahren nach einem der Ansprüche 1-9, wobei das Molverhältnis eines Enzyms des ersten Satzes von Enzymen und eines Enzyms des zweiten Satzes von Enzymen in der Mischung mindestens einen zweifachen Unterschied im Vergleich mit einem Molverhältnis der Enzyme aufweist, die entweder durch die erste oder die zweite Zelllinie als solche exprimiert werden.

11. Verfahren nach einem der Ansprüche 1-9, wobei das Molverhältnis des ersten Satzes von Enzymen, die abgesondert werden, und des zweiten Satzes von Enzymen, die abgesondert werden, mindestens einen zweifachen Unterschied im Vergleich mit einem Molverhältnis der Enzyme aufweist, die entweder durch die erste oder die zweite Zelllinie als solche exprimiert werden.

12. Verfahren nach einem der Ansprüche 1-11, wobei die ersten und zweiten Zelllinien derselbe Stamm sind.

13. Verfahren nach einem der Ansprüche 1-12, wobei die ersten und/oder zweiten Zelllinien Trichoderma reesei-Zelllinien sind.

14. Verfahren nach einem der Ansprüche 1-12, wobei die ersten und/oder zweiten Zelllinien Bacillus-Zelllinien sind.

15. Verfahren nach einem der Ansprüche 1-14, ferner das Bestimmen von Wachstumsprofilen der Zelllinien vor dem Kombinierungsschritt umfassend.

## Revendications

1. Procédé de production d'un mélange d'enzymes catalysant un processus de conversion, comprenant les étapes de:
combinaison d'une première et d'une seconde lignée cellulaire dans un milieu liquide, la première lignée cellulaire codant pour et disposée à exprimer un premier ensemble d'une ou plusieurs enzymes, la seconde lignée cellulaire codant pour et disposée à exprimer un second ensemble d'une ou plusieurs enzymes, le premier et le second ensemble d'enzymes ayant des activités catalytiques améliorant le processus de conversion, un ou plusieurs du premier ensemble d'enzymes étant hétérologues vis-à-vis de la première lignée cellulaire, et un ou plusieurs du second ensemble d'enzymes étant hétérologues vis-à-vis de la seconde lignée cellulaire;
culture des lignées cellulaires combinées; moyennant quoi les lignées cellulaires sécrètent les enzymes dans le milieu, ou sont lysées libérant les enzymes, fournissant ainsi un mélange d'enzymes en proportions efficaces pour améliorer le processus de conversion,
où la première et la seconde lignée cellulaire sont des lignées cellulaires de champignons filamenteux ou des lignées cellulaires bactériennes.

2. Procédé selon la revendication 1, dans lequel un ou plusieurs du second ensemble d'enzymes ne sont pas codés par la première lignée cellulaire; ou
un ou plusieurs du second ensemble d'enzymes sont exprimés à un niveau inférieur par la première lignée cellulaire.

3. Procédé selon la revendication 1, comprenant en outre:
l'identification d'une pluralité d'enzymes catalysant le processus de conversion;
l'identification ou la construction d'une première lignée cellulaire codant pour, et disposée à exprimer un premier ensemble d'une ou plusieurs des enzymes identifiées, et une seconde lignée cellulaire codant pour, et disposée à exprimer un second ensemble d'une ou plusieurs enzymes pour fournir la première et la seconde lignée cellulaire.

4. Procédé selon la revendication 1, comprenant en outre
l'identification ou la construction de lignées cellulaires codant pour et disposées à exprimer différents ensembles d'enzymes catalysant un processus de conversion, les lignées cellulaires ayant été cultivées sous des conditions de pression sélectives ou sous des conditions qui permettent la croissance auxotrophe afin de conserver leur disposition à exprimer les enzymes pour former une banque de lignées cellulaires; où la pluralité des lignées cellulaires comprend la première lignée cellulaire codant pour et disposée à exprimer un premier ensemble d'une ou plusieurs enzymes, et la seconde lignée cellulaire codant pour et disposée à exprimer un second ensemble d'une ou plusieurs enzymes;
la sélection de la première et de la seconde lignée cellulaire de la banque.

5. Procédé selon la revendication 4, dans lequel les lignées cellulaires sont cultivées sous différentes pressions sélectives dans l'étape d'identification et sans pression sélective dans l'étape de culture.

6. Procédé de préparation d'une banque de cellules, comprenant
l'identification d'une pluralité d'enzymes catalysant un processus de conversion; l'identification ou la construction d'une pluralité de lignées cellulaires codant pour et disposées à exprimer différents ensembles de la pluralité d'enzymes;
la propagation des lignées cellulaires sous différentes pressions sélectives ou sous des conditions qui permettent la croissance auxotrophe pour conserver la disposition à exprimer l'ensemble d'enzymes codées par une lignée cellulaire pour fournir une banque de cellules; et
la combinaison de différentes combinaisons des lignées cellulaires, la culture des lignées cellulaires combinées dans un milieu liquide, où les enzymes sont sécrétées ou les cellules sont lysées et les enzymes libérées vers le milieu pour fournir des mélanges différents d'enzymes, et la comparaison de la capacité des enzymes à améliorer le processus de conversion,
où la pluralité des enzymes sont hétérologues vis-à-vis de la pluralité des cellules,
et où la pluralité des lignées cellulaires sont des lignées cellulaires de champignons filamenteux ou des lignées cellulaires bactériennes.

7. Procédé selon l'une quelconque des revendications 1 à 6, comprenant en outre la séparation du mélange d'enzymes des cellules dans la culture.

8. Procédé selon l'une quelconque des revendications 1 à 7, comprenant en outre la combinaison du mélange d'enzymes avec un substrat, le mélange d'enzymes améliorant la conversion du substrat en un produit.

9. Procédé selon l'une quelconque des revendications 1 à 8, dans lequel le substrat est la cellulose et/ou l'hémicellulose et le produit est le glucose, ou le substrat est l'amidon et le produit est un sucre.

10. Procédé selon l'une quelconque des revendications 1 à 9, dans lequel le rapport molaire d'une enzyme du premier ensemble d'enzymes et d'une enzyme du second ensemble d'enzymes dans le mélange est différent d'au moins deux fois celui d'un rapport molaire des enzymes exprimées par soit la première soit la seconde lignée cellulaire seule.

11. Procédé selon l'une quelconque des revendications 1 à 9, dans lequel le rapport molaire du premier ensemble d'enzymes qui sont sécrétées et du second ensemble d'enzymes qui sont sécrétées est au moins deux fois différent d'un rapport molaire des enzymes exprimées soit par la première soit la seconde lignée cellulaire seule.

12. Procédé selon l'une quelconque des revendications 1 à 11, dans lequel la première et la seconde lignée cellulaire sont la même souche.

13. Procédé selon l'une quelconque des revendications 1 à 12, dans lequel la première et/ou la seconde lignée cellulaire sont des lignées cellulaires de Trichoderma reesei.

14. Procédé selon l'une quelconque des revendications 1 à 12, dans lequel la première et/ou la seconde lignée cellulaire sont des lignées cellulaires du genre Bacillus.

15. Procédé selon l'une quelconque des revendications 1 à 14, comprenant en outre la détermination des profils de croissance des lignées cellulaires avant l'étape de combinaison.
